# EUROPEAN PATENT APPLICATION

(11) **EP 3 553 078 A1**
(43) Date of publication of application: **16.10.2019**
(21) Application number: 18166787.4
(22) Date of filing: 11.04.2018
(51) Int. Cl.: C07K 14/54

(54) **GLYCO-ENGINEERED INTERLEUKIN-4 BASED ANTAGONISTS**

(71) Applicant: Julius-Maximilians-Universität Würzburg, 97070 Würzburg (DE)
(72) Inventor: MÜLLER, Thomas Dieter, 97209 Veitshöchheim (DE); SARAH K., Thomas, 97072 Würzburg (DE); FIEBIG, Juliane E., 97084 Würzburg (DE)
(74) Representative: Schiweck Weinzierl Koch Patentanwälte Partnerschaft mbB

(57) **Abstract**

The present invention relates to IL-4 muteins capable of inhibiting IL-4 and/or IL-13 signalling, wherein a non-protein-polymer is conjugated to the mutein via a mutated amino acid, which allows site-directed conjugation of a non-protein polymer. The present invention further relates to nucleic acids and vectors encoding the IL-4 muteins as well as pharmaceutical compositions, kit of parts comprising the IL-4 muteins. The invention further relates to the IL-4 muteins for use in methods of treatment.

SEQUENCE LISTING

<110>  Julius-Maximilians-Universitaet Wuerzburg

<120>  GLYCO-ENGINEERED INTERLEUKIN-4 BASED ANTAGONISTS

<130>  JMU16220EP

<160>  30

<170>  PatentIn version 3.5

<210>  1
<211>  129
<212>  PRT
<213>  Homo sapiens

<400>  1

<210>  2
<211>  129
<212>  PRT
<213>  Artificial

<220>
<223>  K117C F82D IL-4 mutein

<400>  2

His Lys Cys Asp Ile Thr Leu Gln Glu Ile Ile Lys Thr Leu Asn Ser
1               5                   10                  15

Leu Thr Glu Gln Lys Thr Leu Cys Thr Glu Leu Thr Val Thr Asp Ile
            20                  25                  30

Phe Ala Ala Ser Lys Asn Thr Thr Glu Lys Glu Thr Phe Cys Arg Ala
        35                  40                  45

Ala Thr Val Leu Arg Gln Phe Tyr Ser His His Glu Lys Asp Thr Arg
    50                  55                  60

Cys Leu Gly Ala Thr Ala Gln Gln Phe His Arg His Lys Gln Leu Ile
65                  70                  75                  80

Arg Asp Leu Lys Arg Leu Asp Arg Asn Leu Trp Gly Leu Ala Gly Leu
                85                  90                  95

Asn Ser Cys Pro Val Lys Glu Ala Asn Gln Ser Thr Leu Glu Asn Phe
            100                 105                 110

Leu Glu Arg Leu Cys Thr Ile Met Arg Glu Lys Tyr Ser Lys Cys Ser
        115                 120                 125

Ser

<210>  3
<211>  129
<212>  PRT
<213>  Artificial

<220>
<223>  K117C F82D N38C IL-4 mutein

<400>  3

His Lys Cys Asp Ile Thr Leu Gln Glu Ile Ile Lys Thr Leu Asn Ser
1               5                   10                  15

Leu Thr Glu Gln Lys Thr Leu Cys Thr Glu Leu Thr Val Thr Asp Ile
            20                  25                  30

Phe Ala Ala Ser Lys Cys Thr Thr Glu Lys Glu Thr Phe Cys Arg Ala
        35                  40                  45

Ala Thr Val Leu Arg Gln Phe Tyr Ser His His Glu Lys Asp Thr Arg
    50                  55                  60

Cys Leu Gly Ala Thr Ala Gln Gln Phe His Arg His Lys Gln Leu Ile
65                  70                  75                  80

Arg Asp Leu Lys Arg Leu Asp Arg Asn Leu Trp Gly Leu Ala Gly Leu
                85                  90                  95

Asn Ser Cys Pro Val Lys Glu Ala Asn Gln Ser Thr Leu Glu Asn Phe
            100                 105                 110

Leu Glu Arg Leu Cys Thr Ile Met Arg Glu Lys Tyr Ser Lys Cys Ser
        115                 120                 125

Ser

<210>  4
<211>  129
<212>  PRT
<213>  Artificial

<220>
<223>  K117C F82D Q20N T28N K61N IL-4 mutein

<400>  4

His Lys Cys Asp Ile Thr Leu Gln Glu Ile Ile Lys Thr Leu Asn Ser
1               5                   10                  15

Leu Thr Glu Asn Lys Thr Leu Cys Thr Glu Leu Asn Val Thr Asp Ile
            20                  25                  30

Phe Ala Ala Ser Lys Asn Thr Thr Glu Lys Glu Thr Phe Cys Arg Ala
        35                  40                  45

Ala Thr Val Leu Arg Gln Phe Tyr Ser His His Glu Asn Asp Thr Arg
    50                  55                  60

Cys Leu Gly Ala Thr Ala Gln Gln Phe His Arg His Lys Gln Leu Ile
65                  70                  75                  80

Arg Asp Leu Lys Arg Leu Asp Arg Asn Leu Trp Gly Leu Ala Gly Leu
                85                  90                  95

Asn Ser Cys Pro Val Lys Glu Ala Asn Gln Ser Thr Leu Glu Asn Phe
            100                 105                 110

Leu Glu Arg Leu Cys Thr Ile Met Arg Glu Lys Tyr Ser Lys Cys Ser
        115                 120                 125

Ser

<210>  5
<211>  129
<212>  PRT
<213>  Artificial

<220>
<223>  K117C F82D N38C Y124N IL-4 mutein

<400>  5

His Lys Cys Asp Ile Thr Leu Gln Glu Ile Ile Lys Thr Leu Asn Ser
1               5                   10                  15

Leu Thr Glu Gln Lys Thr Leu Cys Thr Glu Leu Thr Val Thr Asp Ile
            20                  25                  30

Phe Ala Ala Ser Lys Cys Thr Thr Glu Lys Glu Thr Phe Cys Arg Ala
        35                  40                  45

Ala Thr Val Leu Arg Gln Phe Tyr Ser His His Glu Lys Asp Thr Arg
    50                  55                  60

Cys Leu Gly Ala Thr Ala Gln Gln Phe His Arg His Lys Gln Leu Ile
65                  70                  75                  80

Arg Asp Leu Lys Arg Leu Asp Arg Asn Leu Trp Gly Leu Ala Gly Leu
                85                  90                  95

Asn Ser Cys Pro Val Lys Glu Ala Asn Gln Ser Thr Leu Glu Asn Phe
            100                 105                 110

Leu Glu Arg Leu Cys Thr Ile Met Arg Glu Lys Asn Ser Lys Cys Ser
        115                 120                 125

Ser

<210>  6
<211>  129
<212>  PRT
<213>  Artificial

<220>
<223>  R121C F82D IL-4 mutein

<400>  6

His Lys Cys Asp Ile Thr Leu Gln Glu Ile Ile Lys Thr Leu Asn Ser
1               5                   10                  15

Leu Thr Glu Gln Lys Thr Leu Cys Thr Glu Leu Thr Val Thr Asp Ile
            20                  25                  30

Phe Ala Ala Ser Lys Asn Thr Thr Glu Lys Glu Thr Phe Cys Arg Ala
        35                  40                  45

Ala Thr Val Leu Arg Gln Phe Tyr Ser His His Glu Lys Asp Thr Arg
    50                  55                  60

Cys Leu Gly Ala Thr Ala Gln Gln Phe His Arg His Lys Gln Leu Ile
65                  70                  75                  80

Arg Asp Leu Lys Arg Leu Asp Arg Asn Leu Trp Gly Leu Ala Gly Leu
                85                  90                  95

Asn Ser Cys Pro Val Lys Glu Ala Asn Gln Ser Thr Leu Glu Asn Phe
            100                 105                 110

Leu Glu Arg Leu Lys Thr Ile Met Cys Glu Lys Tyr Ser Lys Cys Ser
        115                 120                 125

Ser

<210> 7
<211> 129
<212> PRT
<213> Artificial

<220>
<223> R121C F82D Q20N T28N K61N IL-4 mutein

<400> 7

His Lys Cys Asp Ile Thr Leu Gln Glu Ile Ile Lys Thr Leu Asn Ser
1               5                   10                  15

Leu Thr Glu Asn Lys Thr Leu Cys Thr Glu Leu Asn Val Thr Asp Ile
            20                  25                  30

Phe Ala Ala Ser Lys Asn Thr Thr Glu Lys Glu Thr Phe Cys Arg Ala
        35                  40                  45

Ala Thr Val Leu Arg Gln Phe Tyr Ser His His Glu Asn Asp Thr Arg
    50                  55                  60

Cys Leu Gly Ala Thr Ala Gln Gln Phe His Arg His Lys Gln Leu Ile
65                  70                  75                  80

Arg Asp Leu Lys Arg Leu Asp Arg Asn Leu Trp Gly Leu Ala Gly Leu
                85                  90                  95

Asn Ser Cys Pro Val Lys Glu Ala Asn Gln Ser Thr Leu Glu Asn Phe
            100                 105                 110

Leu Glu Arg Leu Lys Thr Ile Met Cys Glu Lys Tyr Ser Lys Cys Ser
        115                 120                 125

Ser

<210>  8
<211>  129
<212>  PRT
<213>  Artificial

<220>
<223>  R121C F82D N38C IL-4 mutein

<400>  8

His Lys Cys Asp Ile Thr Leu Gln Glu Ile Ile Lys Thr Leu Asn Ser
1               5                   10                  15

Leu Thr Glu Gln Lys Thr Leu Cys Thr Glu Leu Thr Val Thr Asp Ile
            20                  25                  30

Phe Ala Ala Ser Lys Cys Thr Thr Glu Lys Glu Thr Phe Cys Arg Ala
        35                  40                  45

Ala Thr Val Leu Arg Gln Phe Tyr Ser His His Glu Lys Asp Thr Arg
    50                  55                  60

Cys Leu Gly Ala Thr Ala Gln Gln Phe His Arg His Lys Gln Leu Ile
65                  70                  75                  80

Arg Asp Leu Lys Arg Leu Asp Arg Asn Leu Trp Gly Leu Ala Gly Leu
                85                  90                  95

Asn Ser Cys Pro Val Lys Glu Ala Asn Gln Ser Thr Leu Glu Asn Phe
            100                 105                 110

Leu Glu Arg Leu Lys Thr Ile Met Cys Glu Lys Tyr Ser Lys Cys Ser
        115                 120                 125

Ser

<210>  9

<211>  129
<212>  PRT
<213>  Artificial

<220>
<223>  R121C F82D Q20C T28C N38C K61C IL-4 mutein

<400>  9

His Lys Cys Asp Ile Thr Leu Gln Glu Ile Ile Lys Thr Leu Asn Ser
1               5                   10                  15

Leu Thr Glu Cys Lys Thr Leu Cys Thr Glu Leu Cys Val Thr Asp Ile
            20                  25                  30

Phe Ala Ala Ser Lys Cys Thr Thr Glu Lys Glu Thr Phe Cys Arg Ala
        35                  40                  45

Ala Thr Val Leu Arg Gln Phe Tyr Ser His His Glu Cys Asp Thr Arg
    50                  55                  60

Cys Leu Gly Ala Thr Ala Gln Gln Phe His Arg His Lys Gln Leu Ile
65                  70                  75                  80

Arg Asp Leu Lys Arg Leu Asp Arg Asn Leu Trp Gly Leu Ala Gly Leu
                85                  90                  95

Asn Ser Cys Pro Val Lys Glu Ala Asn Gln Ser Thr Leu Glu Asn Phe
            100                 105                 110

Leu Glu Arg Leu Lys Thr Ile Met Cys Glu Lys Tyr Ser Lys Cys Ser
        115                 120                 125

Ser



<210>  10
<211>  129
<212>  PRT
<213>  Artificial

<220>
<223>  K117C F82D Q20C T28C N38C K61C IL-4 mutein

<400>  10

His Lys Cys Asp Ile Thr Leu Gln Glu Ile Ile Lys Thr Leu Asn Ser
1               5                   10                  15

Leu Thr Glu Cys Lys Thr Leu Cys Thr Glu Leu Cys Val Thr Asp Ile
            20                  25                  30

Phe Ala Ala Ser Lys Cys Thr Thr Glu Lys Glu Thr Phe Cys Arg Ala
        35                  40                  45

Ala Thr Val Leu Arg Gln Phe Tyr Ser His His Glu Cys Asp Thr Arg
    50                  55                  60

Cys Leu Gly Ala Thr Ala Gln Gln Phe His Arg His Lys Gln Leu Ile
65                  70                  75                  80

Arg Asp Leu Lys Arg Leu Asp Arg Asn Leu Trp Gly Leu Ala Gly Leu
                85                  90                  95

Asn Ser Cys Pro Val Lys Glu Ala Asn Gln Ser Thr Leu Glu Asn Phe
            100                 105                 110

Leu Glu Arg Leu Cys Thr Ile Met Arg Glu Lys Tyr Ser Lys Cys Ser
        115                 120                 125

Ser

<210>  11
<211>  24
<212>  DNA
<213>  Artificial

<220>
<223>  Primer R121D Y124D_s

<400>  11
gacgatcatg gacgagaaag attc                                              24

<210>  12
<211>  24
<212>  DNA
<213>  Artificial

<220>
<223>  Primer R121D Y124D_as

<400>  12
gaatctttct cgtccatgat cgtc                                              24

<210>  13
<211>  24
<212>  DNA
<213>  Artificial

<220>
<223>  Primer F82D_s

<400>  13
cagctgatcc gagacctgaa acgg                                              24

<210>  14
<211>  23
<212>  DNA
<213>  Artificial

<220>
<223>  Primer F82D_as

<400>  14
cgtttcaggt ctcggatcag ctg                                              23


<210>  15
<211>  26
<212>  DNA
<213>  Artificial

<220>
<223>  Primer N38C_s

<400>  15
ctgcctccaa gtgcacaact gagaag                                           26


<210>  16
<211>  26
<212>  DNA
<213>  Artificial

<220>
<223>  Primer N38C_as

<400>  16
cttctcagtt gtgcacttgg aggcag                                           26


<210>  17
<211>  37
<212>  DNA
<213>  Artificial

<220>
<223>  Primer R121C_s

<400>  17
ggctaaagac gatcatgtgt gagaaatatt caaagtg                               37


<210>  18
<211>  37
<212>  DNA
<213>  Artificial

<220>
<223>  R121C_as

<400>  18
cactttgaat atttctcaca catgatcgtc tttagcc                               37


<210>  19
<211>  30

<212>  DNA
<213>  Artificial

<220>
<223>  Primer Q20N_s

<400>  19
gcctcacaga gaacaagact ctgtgcaccg                                        30


<210>  20
<211>  30
<212>  DNA
<213>  Artificial

<220>
<223>  Primer Q20N_as

<400>  20
cggtgcacag agtcttgttc tctgtgaggc                                        30


<210>  21
<211>  23
<212>  DNA
<213>  Artificial

<220>
<223>  Primer T28N_s

<400>  21
gcaccgagtt gaacgtaaca gac                                               23


<210>  22
<211>  23
<212>  DNA
<213>  Artificial

<220>
<223>  Primer T28N_as

<400>  22
gtctgttacg ttcaactcgg tgc                                               23


<210>  23
<211>  25
<212>  DNA
<213>  Artificial

<220>
<223>  Primer K61N_s

<400>  23
ccaccatgag aacgacactc gctgc                                             25


<210>  24
<211>  25
<212>  DNA
<213>  Artificial

<220>
<223>  K61N_as

<400>  24
gcagcgagtg tcgttctcat ggtgg                                          25


<210>  25
<211>  36
<212>  DNA
<213>  Artificial

<220>
<223>  Primer K117N_T118A_ I119T_s

<400>  25
ggaaaggcta aacgcgacca tgagagagaa atattc                              36


<210>  26
<211>  36
<212>  DNA
<213>  Artificial

<220>
<223>  Primer K117N_T118A_ I119T_as

<400>  26
gaatatttct ctctcatggt cgcgtttagc ctttcc                              36


<210>  27
<211>  38
<212>  DNA
<213>  Artificial

<220>
<223>  Primer R121N_K123S_s

<400>  27
ctaaagacga tcatgaacga gagctattca aagtgttc                            38


<210>  28
<211>  37
<212>  DNA
<213>  Artificial

<220>
<223>  Primer R121N_K123S_as

<400>  28
gaacactttg aatagctctc gttcatgatc gtcttag                             37


<210>  29
<211>  26
<212>  DNA
<213>  Artificial

<220>
<223>  Primer N38Q_s

<400>  29
ctgcctccaa gcagacaact gagaag                                          26

<210>  30
<211>  26
<212>  DNA
<213>  Artificial

<220>
<223>  Primer N38Q_as

<400>  30
cttctcagtt gtctgcttgg aggcag                                          26

## Description

### TECHNICAL FIELD OF THE INVENTION

The present invention relates to IL-4 muteins capable of inhibiting IL-4 and/or IL-13 signalling, wherein a non-protein-polymer is conjugated to the mutein via a mutated amino acid, which allows site-directed conjugation of a non-protein polymer. The present invention further relates to nucleic acids and vectors encoding the IL-4 muteins as well as pharmaceutical compositions, kit of parts comprising the IL-4 muteins. The invention further relates to the IL-4 muteins for use in methods of treatment.

### BACKGROUND

The 129 amino acid cytokine Interleukin (IL)-4 is produced by various hematopoietic cells and exerts multiple effects in the immune system. One major function is in the regulation of T-helper cell differentiation, where it triggers the development of uncommitted naive CD4⁺ T-helper cells (Th₀) into subtype 2 T-helper cells (Th₂). Mature Th₂ cells secrete a specific subset of cytokines including IL-4, IL-5 and IL-13 that mediate the inflammatory immune response directed against extracellular pathogens (Abbas et al. 1996).

By secreting IL-4, Th₂-cells suppress the development of Th₁-phenotype thereby maintaining Th₂ proliferation and activity (Parronchie et al. 1992, Nakamura et al. 1997). Physiologic effects of IL-4 and IL-13 partially coincide. Both cytokines induce upregulation of vascular cell adhesion molecule-1 (VCAM1) on endothelial cells and thus facilitate leucocyte migration from the blood to the site of infection (Bochner et al. 1995, Schleimer et al. 1992). Pathophysiologically most important however, IL-4 and IL-13 drive immunoglobulin class switching in B-cells to produce antibodies of the immunoglobulin E (IgE) class (Punnonen et al. 1993, Worm et al. 1997). In addition, IL-4 and IL-13 up-regulate Fcε-receptors for IgE immunoglobulins on mast cells, eosinophils and B-cells thereby enhancing IgE dependent responses (Xia et al. 1997, Kaur et al. 2006). IL-4/IL-13 mediated IgE sensitization is of great pathophysiological significance, as IgE antibodies play a major role in the onset and progression of various atopic diseases, such as asthma, atopic dermatitis and allergic rhinitis. The recognition of a specific antigen/allergen through the IgE/Fcε complex on mast cells as well as basophilic and eosinophilic granulocytes stimulates degranulation of the cell leading to the release of pro-inflammatory mediators, like the anticoagulant heparin and the vasodilator histamine, which promote transport of immune cells to the site of infection. In addition, prostaglandins, leukotrienes and proteases released upon degranulation facilitate local inflammation by causing muscle constriction and tissue remodeling (Stone et al. 2010). However, there are also some functional differences between IL-4 and IL-13. Because human T-cells predominantly express functional IL-4 type I receptor on their cell surface (Graber et al. 1998), IL-13 does not appear to play a role in Th₂-cell polarization but rather seems to be important for the inflammatory effector phase. In particular, IL-13 induces increased mucus production and is more strongly involved in tissue remodeling by causing fibrosis or goblet cell hyperplasia (Zhu et al. 1999, Hershey et al. 2003). Their crucial role in the pathophysiology of atopic diseases has made IL-4 and IL-13 key targets for novel therapeutic approaches to fight allergies and asthma.

IL-4 and IL-13 share a highly overlapping set of transmembrane receptors, which upon ligand-mediated activation induce the Jak/STAT signaling cascade. IL-4 can form two types of heterodimeric receptor assemblies, termed interleukin-4 type I and type II receptor. In a sequential interaction process IL-4 binds first to its high affinity receptor IL-4 receptor α (IL-4Rα, CD124) and this membrane-located complex then recruits either one of two other receptors, which is either the so-called common γ chain (short: yc; CD132) to form the IL-4 type-I receptor, or IL-13 receptor IL-13Rα1 (CD213a1) to form the IL-4 type-II receptor (LaPorte et al. 2008). The type I receptor is activated exclusively by IL-4, although the common γ chain itself is shared by other cytokine receptors, e.g. IL-2, IL-7, IL-9, IL-15 and IL-21 (Leonard et al. 2001). The IL-4 type II receptor can be activated by IL-4 and IL-13 and hence both cytokines assemble and activate the same receptor complex possibly explaining their overlapping functions in vivo. However, IL-13 induces receptor assembly in a reversed order, where IL-13 first binds to its high affinity receptor IL-13Rα1 before engaging IL-4Rα (LaPorte et al. 2008, Andrews et al. 2002).

The overlapping receptor usage of IL-4 and IL-13 is a distinct feature that allows simultaneous blocking of downstream signaling of both cytokines by specifically targeting the receptor IL-4Rα. Therapeutics acting in this manner have been established. Using the classical route to interfere with signaling of extracellular ligand-receptor systems, monoclonal anti-IL-4Rα antibodies, AMG317 (Amgen) and Dupilumab (Regeneron/Sanofi), have been developed, which bind IL-4Rα, thereby neutralizing IL-4 and IL-13 signaling (Gandhi et al. 2017). Dupilumab has been recently approved for treatment of atopic dermatitis and is currently being tested in clinical trials for treatment of allergic asthma, thereby confirming that IL-4Rα is indeed an effective therapeutic target for atopic diseases (Barranco et al. 2017). A second, but not antibody-based strategy to inhibit IL-4 and IL-13 signaling has been implemented in the past by employing a mechanistically highly similar approach as for Dupilumab. From a mutagenesis study two positions in IL-4 were shown to convert the IL-4 protein into a highly effective antagonist that binds to IL-4Rα with wildtype-like affinity but is incapable to activate either the type I or the type II receptor as it cannot bind to γc or IL-13Rα1 (Duschl et al. 1995, Tony et al 1994). This IL-4 double variant harboring the mutations R121D and Y124D was termed Pitrakinra. It was tested for application in allergic asthma until clinical phase trial Ila, where it failed to meet the endpoint criteria, although positive therapeutical effects, e.g. fewer number of allergen induced exacerbations could be observed (Wenzel et al. 2007). Due to failure to meet the endpoint criteria, further developments of Pitrakinra were stopped. Some of the disadvantages were the very short half-life of Pitrakinra in vivo due its small size and lack of glycosylation (Longphre et al. 2010), which was a result of its production in prokaryotic expression systems. To overcome the short half-life, PEGylation of Pitrakinra was tested, however, even though coupling of the PEG moiety was done site-specific, the modification significantly impaired efficacy of the IL-4 inhibitor (WO 2011/106779). Accordingly, there is still a need for improved IL-4 and/or IL-13 antagonists, which have a higher stability towards proteolytic degradation. The technical problem therefore is to comply with this need.

### SUMMARY OF THE INVENTION

The technical problem is solved by the subject-matter as defined in the claims. It is presented herein an improved version of Pitrakinra, which evades its inherent disadvantages using a novel approach to simultaneously implement IL-4 antagonism and enhance pharmacological properties to ensure a significant half-life *in vivo.*

Accordingly, the present invention relates in a first aspect to an IL-4 mutein capable of inhibiting IL-4 and/or IL-13 signalling, wherein the IL-4 mutein comprises an amino acid mutation at each of sequence positions 82 and 121 in comparison to the human mature IL-4 (positions 25-153 of UniProt database entry P05112, SEQ ID NO: 1), wherein the amino acid at sequence position 82 is mutated to aspartic acid and the amino acid at sequence position 121 is mutated to an amino acid, which allows site-directed conjugation of a non-protein-polymer, and wherein a non-protein-polymer is conjugated to the mutein via the mutated amino acid at sequence position 121.

The present invention further relates in a second aspect to an IL-4 mutein capable of inhibiting IL-4 and/or IL-13 signalling, wherein the IL-4 mutein comprises an amino acid mutation at each of sequence positions 82 and 117 in comparison to the human mature IL-4 (positions 25-153 of UniProt database entry P05112, SEQ ID NO: 1), wherein the amino acid at position 82 is mutated to aspartic acid and the amino acid at position 117 is mutated to an amino acid, which allows site-directed conjugation of a non-protein-polymer, and wherein a non-protein-polymer is conjugated to the IL-4 mutein via the mutated amino acid of position 117.

Preferably, the mutein of the second aspect is an antagonist of IL-4 receptor type II and/or preferably comprises a further mutation at position 124, wherein the amino acid at position 124 is mutated to an amino acid selected from the group consisting of glycine, lysine and asparagine.

The IL-4 mutein of the first and the second aspect preferably further comprises at least one, two or three mutated amino acid(s) at a sequence position selected from the group consisting of sequence positions 20, 28 and 61, preferably providing one additional glycosylation site at each mutated sequence position, more preferably the IL-4 mutein comprises asparagine at each mutated sequence position selected from the group consisting of sequence positions 20, 28 and 61 and most preferably, the IL-4 mutein is N-glycosylated at at least one sequence position selected from the group consisting of sequence positions 20, 28 and 61.

Alternatively, the present invention relates to the IL-4 mutein of the first and the second aspect further comprising at least one, two or three mutated amino acid(s) at a sequence position selected from the group consisting of sequence positions 20, 28 and 61, wherein preferably the at least one, two or three mutated amino acid(s) at a sequence position selected from the group consisting of sequence positions 20, 28 and 61 is mutated to an amino acid, which allows site-direct conjugation of a non-protein polymer and wherein a non-protein polymer is conjugated to the IL-4 mutein via the mutated amino acid at a sequence position selected from the group consisting of positions 20, 28 and 61.

The IL-4 mutein of the first and the second aspect preferably further comprises a mutated amino acid at sequence position 38, wherein the amino acid at sequence position 38 is mutated to an amino acid, which allows site-directed conjugation of a non-protein-polymer and wherein a non-protein polymer is conjugated to the IL-4 mutein via the mutated amino acid of sequence position 38.

The IL-4 mutein of the first and the second aspect preferably comprises, or more preferably consists of/has an amino acid sequence with at least 75 %, at least 80 %, at least 85 %, at least 90 %, at least 95 % or at least 99 % sequence identity to an amino acid sequence selected from the group consisting of SEQ ID NOs: 2-10, wherein the IL-4 mutein comprises at least one amino acid, which allows site-directed conjugation of a non-protein polymer and wherein a non-protein polymer is conjugated to the IL-4 mutein via the amino acid, which allows site-directed conjugation of a non-protein polymer. More preferably, the IL-4 mutein of the first and the second aspect has or consists of an amino acid sequence selected from the group consisting of SEQ ID NOs: 2-10, wherein the IL-4 mutein comprises at least one amino acid, which allows site-directed conjugation of a non-protein polymer and wherein a non-protein polymer is conjugated to the IL-4 mutein via the amino acid, which allows site-directed conjugation of a non-protein polymer.

The amino acid, which allows site-directed conjugation of a non-protein-polymer, preferably is cysteine.

The non-protein polymer preferably comprises a mono- or oligosaccharide, polysialic acid, or a complex N-glycan-like oligosaccharide structure.

The non-protein polymer preferably is chemically modified to allow conjugation to an amino acid, which allows site-directed conjugation of a non-protein polymer, more preferably, the non-protein polymer comprises a thiol group or is coupled to SMCC (succinimidyl 4-(N-maleimidomethyl)cyclohexane-1-carboxylate).

The IL-4 mutein preferably has a glycosylation pattern obtained by an expression in a eukaryotic cell, wherein the glycosylation pattern more preferably is obtained by expression in a mammalian cell.

The present invention further relates to a nucleic acid encoding the IL-4 mutein of the invention.

The present invention further relates to a vector comprising the nucleic acid of the invention; the vector preferably further comprises a promoter operatively linked to the nucleic acid.

The present invention further relates to a host cell comprising the nucleic acid or the vector of the invention. The host cell preferably is eukaryotic, more preferably mammalian.

The present invention further relates to a method for producing an IL-4 mutein according to any of the first and the second aspect, comprising the steps of: (a) culturing the host cell of claim 23 or 24 under conditions allowing the production of the IL-4 mutein, (b) obtaining the IL-4 mutein, (c) optionally conjugating the IL-4 mutein with a non-protein polymer, (d) optionally removing non-conjugated IL-4 muteins, and (e) optionally enzymatic elongation of chemically coupled mono- and/or oligosaccharides.

The present invention further relates to a pharmaceutical composition comprising the IL-4 mutein of the first and the second aspect, wherein the pharmaceutical composition preferably is adapted for inhalation or subcutaneous injection.

The present invention further relates to a pharmaceutical kit of parts comprising the pharmaceutical composition of the invention and an anti-inflammatory agent.

The present invention further relates to the IL-4 muteins of the first and the second aspect, the pharmaceutical composition of any of the invention or the pharmaceutical kit of parts of the invention for use in a method of treating a subject.

The present invention further relates to the IL-4 muteins of the first and the second aspect, the pharmaceutical composition of any of the invention or the pharmaceutical kit of parts of the invention for use in a method of treating an inflammatory disease, wherein the inflammatory disease preferably is selected from the list consisting of asthma, arthritis, colitis ulcera, atopic dermatitis, allergy and allergic asthma, or leishmaniosis. The subject preferably is human.

### BRIEF DESCRIPTION OF THE DRAWINGS

The invention will be better understood with reference to the detailed description when considered in conjunction with the non-limiting examples and the accompanying drawings, in which:

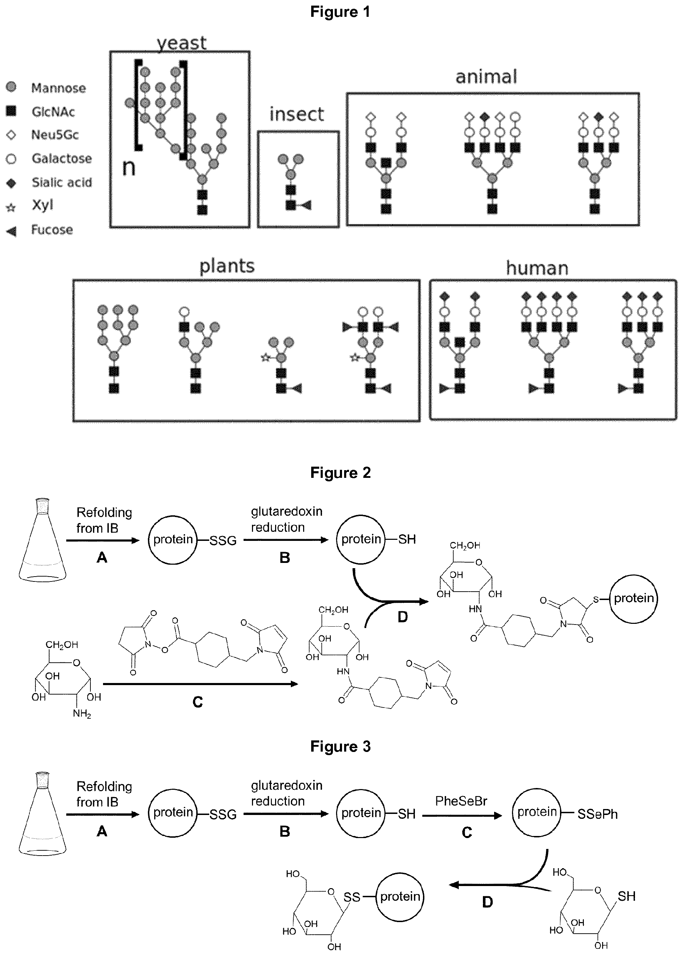

**Fig. 1** shows typical glycosylation patterns of proteins that are attached to in various kingdoms, phyla, classes and species of eukaryotes. These patterns are examples for non-protein polymers that may be conjugated to the IL-4 mutein of the invention. The conjugation may occur biosynthetically, i.e. by expression in the described kingdoms, phyla, classes and species of eukaryotes, or chemically, preferably by activating the non-protein polymer as described herein.
**Fig. 2** shows an example of the conjugation of an amino sugar to a reduced IL-4 mutein via the bifunctional cross-linker SMCC. A) Expression in *E. coli* and oxidative refolding of IL-4 cysteine variants in the presence of a glutathione redox couple B) Enzymatic reduction of the glutathione-mixed disulphide using glutaredoxin-1 yielding the engineered cysteine residue with a free thiol-group C) Reaction of the bifunctional cross-linker SMCC with glucosamine D) Site directed conjugation of the purified SMCC-glucosamine to the free thiol-group of the engineered cysteine residue.
**Fig. 3** shows an example of the conjugation of thiol-glycans to reduced IL-4 muteins using phenylselenylbromide activation. A) Expression in *E. coli* and oxidative refolding of IL-4 cysteine muteins in the presence of a glutathione redox couple B) Enzymatic reduction of the glutathione-mixed disulphide using glutaredoxin-1 yielding the engineered cysteine residue with a free thiol-group C) Activation of the free thiol-group of the engineered cysteine residue with phenylselenylbromide to give the corresponding phenylselenyl sulphide D) Conjugation of the thiol-glycan yielding the disulphide linked protein-glycan conjugate.

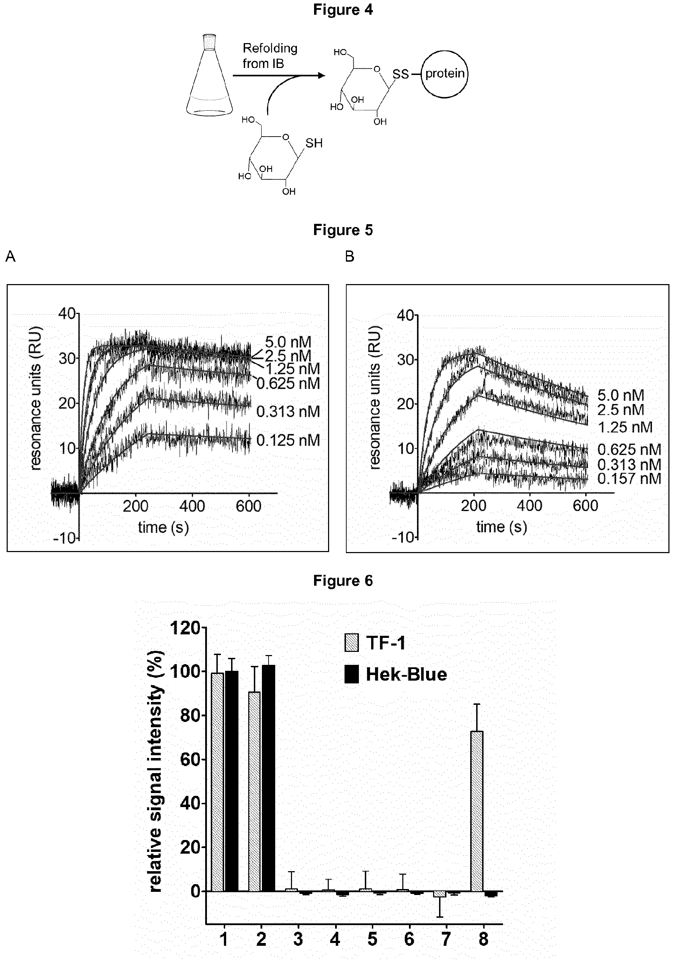

**Fig. 4** shows an example of the conjugation of thiol-glycans to IL-4 muteins during refolding. Expression in *E. coli* and oxidative refolding of IL-4 cysteine muteins in the presence of thiol-glycan.
**Fig. 5A** shows the *in vitro* interaction analysis of the binding of the IL-4 F82D R121C-Glc mutein to IL-4Rα determined by SPR at the indicated concentrations.
**Fig. 5B** shows the *in vitro* interaction analysis of the binding of the IL-4 to IL-4Rα determined by SPR at the indicated concentrations.
**Fig. 6** shows the biologic activity of glyco-engineered IL-4 F82D R121C muteins in HEKBlue and TF-1 cells. Cells were incubated with 50 pM of each variant and signal intensity was normalized to IL-4 WT. Data is shown from three independent experiments (n=3). 1: WT; 2: F82D N38C-Glc; 3: F82D R121C-SMCC-GlcN; 4: F82D R121C-Glc; 5: F82D R121C-4acGlc; 6: F82D R121C-Glc (Hek293); 7: R121D Y124D (Pitrakinra); 8: F82D R121E.

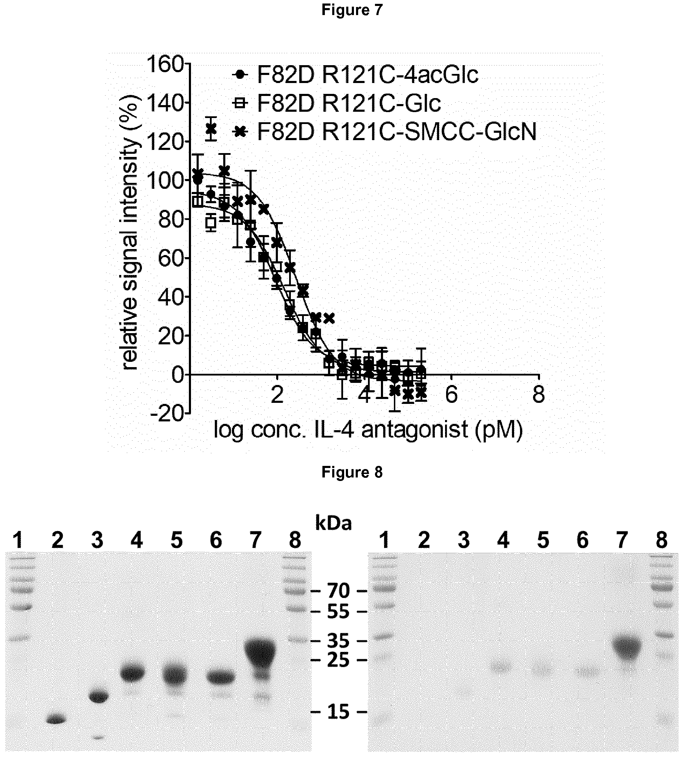

**Fig. 7** shows dose response curves of glyco-engineered IL-4 F82D R121C muteins in TF-1 cells. Cells were incubated with a log 2 titration of the indicated IL-4 F82D R121C muteins in 50 pM IL-4 WT as detailed in the Examples. The signal intensity was normalized to IL-4 WT at 50 pM. Data is shown from three independent experiments (n=3).
**Fig. 8** shows SDS-PAGE of purified Hek293 derived IL-4 hyper glycosylated muteins under non-reducing conditions. The SDS gel was first submitted to periodic acid-Schiff staining (right panel) to visualize the degree of glycosylation, before Coomassie was used for protein detection. 1: protein size standard; 2:F82D N38Q; 3:WT; 4:F82D Q20N; 5:F82D T28N; 6:F82DK61N; 7:F82D Q20N T28N K61N (ConA); 8: marker.

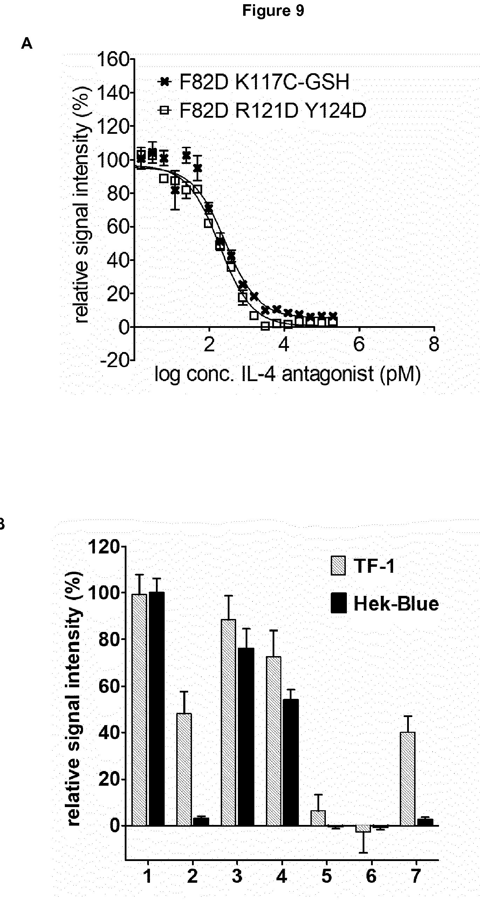

**Fig. 9A** shows the inhibition of IL4-stimulated STAT6 signaling measured by expression of secreted alkaline phosphatase (SEAP) in HEK293 cells (HEKBlue cells, Invitrogen) by IL4 antagonists.
**Fig. 9B** shows the biologic activity of glyco-engineered IL-4 F82D K117C/N variants muteins in HEKBlue and TF-1 cells. Cells were incubated with 50 pM of each variant and signal intensity was normalized to wildtype IL-4. 1:WT; 2: F82D K117C-GSH; 3:F82D K117C-Glc; 4:F82D K117C-4acGlc; 5:F82D N38Q K117N (Hek293); 6:R121D Y124D; 7: F82D N38Q T28N (Hek293).

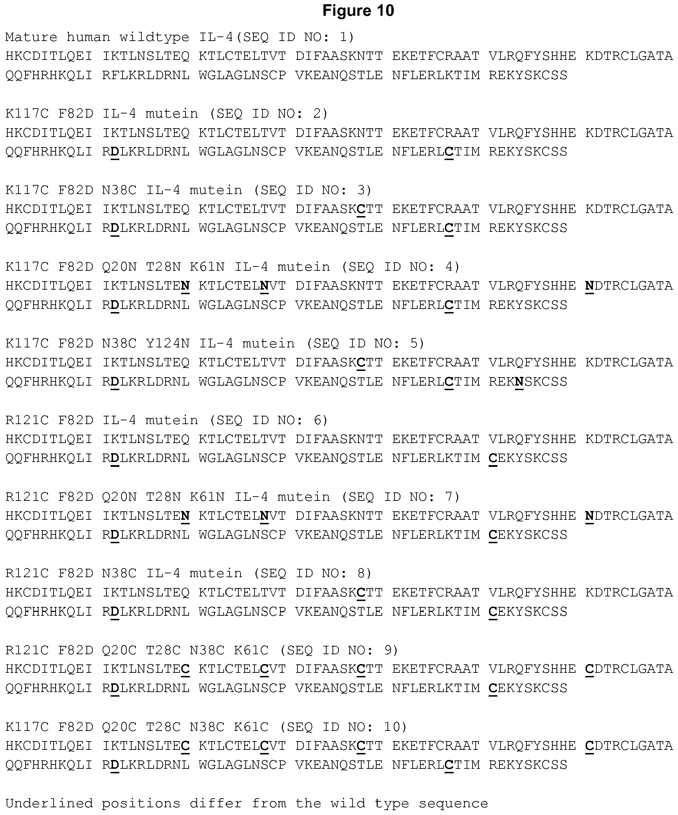

**Fig. 10** shows an alignment of preferred IL-4 mutein sequences and the human wild type IL-4. Underlined sequence positions differ from the wild type IL-4 sequence depicted as SEQ ID NO: 1.

### DETAILED DESCRIPTION OF THE INVENTION

The present invention is described in detail in the following and will also be further illustrated by the appended examples and figures.

Pitrakinra exerts its antagonistic effect through two mutations: arginine 121 and tyrosine 124 are both replaced by aspartate, thereby introducing an electrostatic mismatch in the binding epitopes to the receptors yc and IL-13Rα1 (Muller et al. 1994). While the mutation Y124D already yielded an inactive IL-4, more sensitive cell-based assays showed that the second mutation R121D is required to obtain a highly effective antagonist interfering with both IL-4 and IL-13 signaling (Tony et al. 1994, Kruse et al. 1992, Kruse et al. 1993). The inventors of the present invention surprisingly found that the site-directed attachment of a bulky residue, like e.g. a (branched) sugar moiety, at sequence positions 117 or 121 in the receptor-binding epitope shared by yc and IL13Rα1 renders an IL-4 mutein an IL-4 and/or IL-13 antagonist. All the glyco-engineered IL-4 antagonistic muteins described herein additionally contain the mutation F82D, which was shown to augment the binding affinity of the IL-4 protein to its receptor IL-4Rα by a factor of about 5-fold (Kraich et al. 2006), which will hence benefit the IL-4 muteins, as these might be more effectively compete off endogenous wildtype IL-4 and might render them superior to the original Pitrakinra. Accordingly, preferably all muteins of the invention comprise the mutation F82D.

As used herein, unless otherwise specified, "IL-4" relates to human IL-4. IL-4 is also known as "B-cell stimulatory factor 1 (BSF-1)" or "lymphocyte stimulatory factor 1". Human IL-4 preferably relates to a full-length protein defined by UniProt P05112, a fragment thereof, or a variant thereof. More preferably, human IL-4 refers to positions 25-153 as set forth in SEQ ID NO: 1, which may act as reference scaffold, of the protein defined by UniProt database entry P05112. The numbering for sequence positions used throughout this disclosure relates to the sequence positions in SEQ ID NO: 1 and starts in the UniProt entry P05112 after the signal peptide at histidine 25.

As used herein, a "mutein," a "mutated" entity (whether protein or nucleic acid), or "mutant" refers to the exchange, deletion, or insertion of one or more nucleotides or amino acids, compared to the naturally occurring (wild-type) nucleic acid or protein "reference" scaffold. The term "mutein," as used herein, may also include its functional fragments or variants. Fragments or variants of particular muteins described in the present disclosure preferably retain the function of inhibiting IL-4 and/or IL-13 signaling, and such fragments or variants are "functional fragments or variants" of the reference muteins disclosed herein.

The muteins of the invention are "capable of inhibiting IL-4 and/or IL-13 signaling". "Capable of inhibiting IL-4 and/or IL-13 signaling" as used herein relates to the property of an IL-4 mutein to prevent IL-4 and/or IL-13 induced signaling. Inhibition is this context means a reduction of IL-4 and/or IL-13 activity by, for, example, about 50 % or more, about 60 % or more, about 70 % or more, about 80 % or more, about 90 % or more, about 95 % or more, or about 99 % or more when measured in a suitable activity assay when compared to wild type IL-4 and/or IL-13. While in principle any respective activity assay known to the skilled person can be used, illustrative examples of suitable IL-4 and/or IL-13 activity assays that can be used for determining the inhibitory capability of muteins of the invention are shown in Examples 4, 5 and 8. IL-4 muteins that are capable of inhibiting IL-4 and/or IL-13 signaling may also relate to IL-4 muteins that inhibit the action of IL-4 and/or IL-13 with a Kᵢ of 100 nM or less, 75 nM or less, 50 nM or less or 25 nM or less.

However, "capable of inhibiting IL-4 and/or IL-13 signaling" may also be defined as "being an antagonist of IL-4 receptor type I and/or IL-4 receptor type II". Both, human IL-4 receptor type I and type II comprise IL-4Rα (CD124). The type I receptor further comprises the (common) yc chain and only IL-4 and not IL-13 can bind to IL-4 receptor type I. Here, IL-4 first binds to IL-4Rα and then the second chain, yc (CD132), is associated. The IL-4 type II receptor on contrast interacts with IL-4 and IL-13 and further comprises IL-13Rα1. In case of IL-4, IL-4 first also binds to IL-4Rα but then sequesters IL-13Rα1 (CD213a1). In case of IL-13, IL-13 first binds to IL-13Rα1 and then sequesters IL-4Rα. Accordingly, the IL-4 mutein may be capable of inhibiting IL-4 receptor type I, may be capable of inhibiting IL-4 receptor type II, or may be capable of inhibiting IL-4 receptor type I and IL-4 receptor type II.

Without wishing to be bound by theory, the muteins of the present invention presumably first bind the IL-4Rα chain with high affinity. At sufficiently high concentrations, all IL-4Rα chains are bound to the IL-4 mutein. However, the IL-4 mutein prevents the association of the second part of the IL-4 receptor type I, the yc chain, and/or IL-4 receptor type II, the IL-13Rα1 chain presumably by steric hindrance due to the bulky side chain.

In a first aspect, the present invention relates to an IL-4 mutein capable of inhibiting IL-4 and/or IL-13 signaling, wherein the IL-4 mutein comprises an amino acid mutation at each of sequence positions 82 and 121 in comparison to the human mature IL-4 (which is composed of positions 25-153 of UniProt database entry P05112, SEQ ID NO: 1), wherein the amino acid at sequence position 82 is mutated to aspartic acid and the amino acid at sequence position 121 is mutated to an amino acid, which allows site-directed conjugation of a non-protein-polymer, and wherein a non-protein-polymer is conjugated to the mutein via the mutated amino acid at sequence position 121. In one embodiment, the IL-4 mutein of the first aspect is capable of inhibiting IL-4 and IL-13 signaling. As shown in Examples 4, 5 and 8, the IL-4 mutein of the first aspect is capable of inhibiting IL-4 and/or IL-13 signaling.

In a second aspect, the present invention relates to an IL-4 mutein capable of inhibiting IL-4 and/or IL-13 signaling, wherein the IL-4 mutein comprises an amino acid mutation at each of sequence positions 82 and 117 in comparison to the human mature IL-4 (positions 25-153 of UniProt database entry P05112, SEQ ID NO: 1), wherein the amino acid at position 82 is mutated to aspartic acid and the amino acid at position 117 is mutated to an amino acid, which allows site-directed conjugation of a non-protein-polymer, and wherein a non-protein-polymer is conjugated to the IL-4 mutein via the mutated amino acid of position 117. In one embodiment, the IL-4 mutein of the second aspect is capable of inhibiting IL-4 and IL-13 signaling.

As shown in Example 8, the IL-4 mutein of the second aspect is capable of inhibiting IL-4 and/or IL-13 signaling. However, the ability of the mutein of the second aspect to inhibit IL-13 signaling is dependent on the size of the non-protein-polymer that is conjugated to the IL-4 mutein of the second aspect. The IL-4 mutein with glutathione conjugated to position 117 completely blocks IL-4 receptor type II (as shown by the activity in HEK Blue cells lacking IL-4 receptor type I), whereas it is still able to activate the type I receptor (as shown by the activity in TF-1 cells having both IL-4 receptor type I and type II). The IL-4 muteins K117C-Glc, K117C-4acGlc and K117N Expi (with a complex N-linked glycan at position 117 due to expression in a human host cell) show with increasing size of the non-protein polymer a decrease of IL-4 receptor type II activity (as shown by the activity in HEK Blue cells lacking IL-4 receptor type I). In case of small non-protein-polymers like e.g. a glutathione, glucose or glucose tetraacetate conjugated to the mutein of the second aspect (K117C GSH, K117C Glc, K117C 4ac Glc in Fig. 9B), the mutein may not be able to inhibit signaling derived from the IL-4 receptor type I but may act as (partial) agonist. However, if larger non-protein-polymers like e.g. a complex N-glycan are conjugated to the IL-4 mutein by expression in a eukaryotic host cells, like e.g. HEK293 Expi (see K117N Expi in Fig. 9B) of the second aspect, both IL-4 receptor type I and type II are inhibited. Hence, IL-4 muteins comprising the mutations F82D and K117C or K117N show an increase of antagonism of the IL-4 receptor type II with an increase of size of the non-protein polymer, which is conjugated to the amino acid, which allows site-directed conjugation of a non-protein polymer, at position 117. The inhibitory efficacy is significantly enhanced if the mutein is combined with the mutation T28N, which results in the conjugation of a complex branched N-glycan at position 28 upon production in a human cell line (see also Fig. 9B). In sum, the size and the architecture of the non-protein polymer may affect the inhibitory efficacy as well as the specificity, which IL-4 receptor is blocked.

Accordingly, in one embodiment of the IL-4 mutein of the second aspect, the IL-4 mutein is an antagonist of (only) IL-4 receptor type II. Such an IL-4 mutein preferably comprises a small non-protein-polymer, more preferably a non-protein-polymer with a molecular weight of 250 to 750 Dalton or less. Examples for such small non-protein polymers are glutathione or a trisaccharide, such as maltotriose with a thiol group at the first anomeric C-atom, e.g. Glc-α-Glc-α -Glc- α -SH). Alternatively a branched trisaccharide with the thiol group at the anomeric C-atom of a glucose moiety, which is then conjugated to two other glucose moieties via the OH groups at carbon atoms C2 and C4.

A further mutation of Y124D, Y124G or Y124K in the IL-4 mutein of the second aspect may render the IL-4 mutein of the second aspect an antagonist of both IL-4 receptor type I and type II. This feature is independent of the size of the non-protein-polymer conjugated to the IL-4 mutein via the mutated amino acid of position 117. Accordingly, the present invention relates to an IL-4 mutein of the second aspect, wherein the amino acid at position 124 is mutated to an amino acid selected from the group consisting of glycine, lysine and asparagine.

The muteins of the invention may comprise mutations at further sequence positions. Preferably, the IL-4 muteins of the invention further comprise at least one, at least two, or three mutated amino acid(s) at a sequence position selected from the group consisting of sequence positions 20, 28 and 61. Modifications at these positions, which preferably enable the attachment or conjugation of bulky and/or branched moieties, i.e. of a non-protein polymer, that may further improve the antagonistic effect and/or the proteolytic stability of the IL-4 muteins of the invention.

One possibility to conjugate bulky moieties to the sequence positions 20, 28 and 61 is the introduction of glycosylation sites at each mutated sequence position. If the IL-4 mutein of the invention is expressed in a eukaryotic expression system, the IL-4 mutein will be biosynthetically glycosylated by the host cell. The introduced glycosylation may further inhibit the association of the yc or the IL-13Rα1 chain to the IL-4Rα/IL-4 mutein complex, thereby further inhibiting IL-4 and/or IL-13 signaling, and/or further prevent proteolytic degradation, i.e. increase serum half-life. Accordingly, at least one, at least two or three mutated amino acid(s) at a sequence position selected from the group consisting of sequence positions 20, 28 and 61 provide(s) one additional glycosylation site at each mutated sequence position. Preferably, the IL-4 mutein of the invention comprises asparagine at each mutated sequence position selected from the group consisting of sequence positions 20, 28 and 61. More preferably, the IL-4 mutein is N-glycosylated at at least one sequence position selected from the group consisting of sequence positions 20, 28 and 61. As shown in Table 4, the hyper-glycosylated IL-4 mutein F82D Q20N T28N K61N expressed in the human Hek293 host cell has the highest proteolytic stability, indicating an increased half-life.

Another possibility to conjugate non-protein polymers to the sequence positions 20, 28 and 61 is the mutation to an amino acid, which allows site-directed conjugation of a non-protein polymer and to conjugate a non-protein polymer to a mutated amino acid at sequence position(s) 20, 28, and/or 61. Accordingly the present invention relates to an IL-4 mutein, wherein the at least one, at least two or three mutated amino acid(s) at (a) sequence position(s) selected from the group consisting of sequence positions 20, 28 and 61 of the IL-4 mutein is/are mutated to an amino acid, which allows site-direct conjugation of a non-protein polymer and wherein a non-protein polymer is conjugated to the IL-4 mutein via the mutated amino acid at a sequence position selected from the group consisting of positions 20, 28 and 61.

Further, a bulky moiety, i.e. a non-protein polymer, may be introduced at sequence position 38 of IL-4. This position provides a further possible mutation site for a mutation to an amino acid that allows site-directed conjugation of a non-protein polymer, to which a non-protein polymer may be preferably conjugated. Accordingly, the IL-4 mutein further comprises a mutated amino acid at sequence position 38, wherein the amino acid at sequence position 38 is mutated to an amino acid, which allows site-directed conjugation of a non-protein-polymer and wherein a non-protein polymer is conjugated to the IL-4 mutein via the mutated amino acid of sequence position 38.

The "amino acid, which allows site-directed conjugation of a non-protein polymer" may be an amino acid with a reactive amino acid residue. Reactive amino acid residues in the 20 "standard" natural amino acids include sulfhydryl groups, carboxylic acids, amides, alcohols and amino groups. Preferably, the amino acid, which allows site-directed conjugation of a non-protein polymer, is cysteine. Cysteines have the advantage of a very reactive sulfhydryl side group and are also not very common in proteins. While two cysteines readily build a cysteine bridge in oxidative environments if the two cysteines are in proximity, single cysteines can be interaction partners for non-protein polymers.

Beside the "natural" amino acids, also unnatural amino acids could be envisioned as amino acids, which allow site-directed conjugation of a non-protein polymer. Using genetic code expansion, it is possible to incorporate unnatural amino acids into proteins expressed in prokaryotic and even eukaryotic cells. Serfling et al. 2016 disclosed a method for such an incorporation of a photoactivatable unnatural amino acid into GPCRs. Hence, a person skilled in the art is capable of incorporating an unnatural amino acid into an IL-4 mutein. Examples for suitable unnatural amino acids include, but are not limited to, 3-Azido-L-alanine, 4-Azido-L-homoalanine HCI (L-AHA), 4-Azido-L-phenylalanine 5-Azido-L-ornithine, 5-Azido-L-norvaline, 2-Amino-5-azidopentanoic acid, 6-Azido-L-lysine, 6-Azido-L-lysine and L-Homopropargylglycine (L-HPG).

In another embodiment, the amino acid, which allows site-directed conjugation of a non-protein polymer, may be asparagine, in case it is part of a glycosylation site. A glycosylation site has the consensus sequence Asn-Xaa-Xab, wherein Xaa can be any amino acid except proline and Xab can be Ser or Thr. If necessary, adjacent sequence positions have to be mutated as well to generate the consensus sequence. E.g., to introduce a glycosylation site at position 117 of IL-4, the IL-4 mutein has to carry at least the following mutations: K117N and I119T, preferably additionally T118A. If the amino acid, which allows site-directed conjugation of a non-protein polymer, is asparagine and part of a glycosylation sequence, the conjugation of a non-protein polymer, e.g. a glycan structure, is achieved by expression in eukaryotic host cells as described herein, e.g. in a HEK293 Expi cells to obtain a complex human N-glycan structure (see also Example 8). In one embodiment, the amino acid, which allows site-directed mutagenesis is asparagine, in case it is part of a glycosylation site, or cysteine.

In one embodiment, the amino acid, which allows site-directed conjugation of a non-protein polymer, is not asparagine, aspartic acid, glutamine, glutamic acid and/or lysine. In one embodiment, the amino acid, which allows site-directed conjugation of a non-protein polymer at sequence position 121 is not asparagine, aspartic acid, glutamine, glutamic acid and/or lysine.

The IL-4 mutein may comprise glycosylation sites. Eukaryotic cells detect these signal sequences and attach a specific glycan to the asparagine of the recognition sequence. Mammalian cells are a specific subtype of the domain of eukaryote, which attach significantly different glycan structures, e.g. in comparison to the kingdom of fungi. Accordingly, the present invention relates to an IL-4 mutein having a glycosylation pattern obtained by an expression in a eukaryotic cell, preferably a mammalian cell.

The "non-protein polymer" is a moiety that is suitable for preventing the association of the yc or IL-13Rα1 chain to the complex comprising the IL-4 mutein and IL4-Rα, thereby inhibiting the assembling of the IL-4 receptor type I or type II complex and consequently inhibiting IL-4 and/or IL-13 signalling without being a protein. By using carbohydrates, sugars, glycans or the like, the proteolytic stability of the IL-4 muteins is improved as well. In one embodiment, the non-protein polymer is not polyethylene glycol, polypropylene glycol or a polyoxyalkylene.

The non-protein polymer may comprise a monosaccharide or an oligosaccharide. Monosaccharides are the most basic units of carbohydrates. The general formula is CₙH₂ₙOₙ. Examples of monosaccharides include glucose (dextrose), fructose (levulose) and galactose. Further, each carbon atom that supports a hydroxyl group (so, all of the carbons except for the primary and terminal carbon) is chiral, giving rise to a number of isomeric forms, all with the same chemical formula. For instance, galactose and glucose are both aldohexoses, but have different physical structures and chemical properties. Also derivatives of monosaccharides, which include amino sugars such as galactosamine, glucosamine, sialic acid and N-actecylglucosamine, and sulfosugars such as sulfoquinovose, are within the scope of the invention.

As outlined herein, the non-protein polymer may also comprise an oligosaccharide. An oligosaccharide as used herein is a saccharide polymer containing a small number (typically 2 to 20) of monosaccharides. They are normally present as glycans: oligosaccharide chains linked to lipids or to compatible amino acid side chains in proteins, by N- or O-glycosidic bonds. N-linked oligosaccharides are usually attached to asparagine via a beta linkage to the amine nitrogen of the side chain. However, the non-protein polymer may however be attached to the mutated amino acid, which allows site-directed conjugation of a non-protein polymer, by the use of a cross-linker. Accordingly, the non-protein polymer may also comprise a complex N-glycan-like oligosaccharide structure. Examples for complex N-glycan-like oligosaccharide structures can be found in Figure 1. The oligosaccharide may be modified to allow site-directed conjugation. The oligosaccharide may also comprise an amino group.

The non-protein polymer may also comprise a polysialic acid. Sialic acid is a generic term for the N- or O-substituted derivatives of neuraminic acid, a monosaccharide with a nine-carbon backbone. It is also the name for the most common member of this group, N-acetylneuraminic acid (Neu5Ac or NANA). The amino group preferably bears either an acetyl or a glycosyl group, but other modifications have been described. The hydroxyl substituents may vary considerably; acetyl, lactyl, methyl, sulfate, and phosphate groups are within the scope of the invention. However, the amino group may also be unmodified. Polysialic acid is a carbohydrate comprising α2,8-linked sialic acids and may also be comprised in the non-protein polymer.

The non-protein polymer of the invention preferably is chemically modified to allow conjugation to an amino acid, which allows site-directed conjugation of a non-protein polymer. In one embodiment, the non-protein polymer is modified to contain a thiol group to allow site-directed conjugation. Such a thio-carbohydrate may be directly conjugated to the IL-4 mutein. A glyco-engineering strategy, established by Gamblin et al. 2004 that is termed Glyco-SeS, implements thiol-functionalized carbohydrates for site-directed linker-free conjugation to cysteine residues. Reaction with a phenylselenylating reagent converts the free sulfhydryl group of an engineered cysteine residue into the corresponding mixed phenylselenylsulphide (Bernardes et al. 2006, Gamblin et al. 2004, van Kasteren et al. 2007). Because of the resulting electrophilic nature of the Sulphur atom in the S-Se bond, it readily reacts with a thiol-containing carbohydrate to form a disulphide-linked protein-glycan conjugate. This strategy can also be employed in an alternative setup, where the thio-carbohydrate is first activated with phenylselenylbromide, which can then be coupled to a free unpaired cysteine residue in the protein. Thiol-functionalized carbohydrates in general are monosaccharides, disaccharides or glycans as defined herein, which have been modified to contain at least one, preferably one, sulfhydryl group instead of a hydroxyl group, preferably at the C₁ atom. Examples for such thiol-functionalized carbohydrates and their synthesis are e.g. disclosed in Goncalo et al. (2006), Angew Chem Int Ed, 45(24):4007-4011, and include, but are not limited to, 1-thio-β-D-glucose, 1-thio-mannose, 1-thio-galactose, 1-thio-lactose, 1-thio-maltopentaose, 1-thio- galabiose and 1-thio-β-D-glucose tetraacetate. Another method of synthesizing such functionalized carbohydrates like glycosyl thiols involves the treatment of glycosyl halides with a sulphur nucleophile (either thiourea or potassium thioacetate) in acetone, followed by mild hydrolysis. A further method of synthesizing such functionalized carbohydrates is the reaction of reducing carbohydrates with Lawesson's reagent to thio-glycans (as largest thio-glycan a maltopentaose was shown as example). Given the reaction scheme, the production of thio-glycans is not limited to these mono- and oligosaccharides but should be expandable to also more complex glycan structures as found in natural glycoproteins.

In another embodiment, the non-protein polymer is chemically modified to allow conjugation to an amino acid, which allows site-directed conjugation of a non-protein polymer, preferably by the conjugation to a cross linker, which e.g. is first coupled to an amine of the non-protein polymer. Then, the linker-non-protein polymer-complex may be conjugated to the IL-4 mutein via the amino acid, which allows site-directed conjugation. Such a linker comprises a group, which is reactive towards sulfhydryl groups, like e.g. haloacetyls, maleimides, aziridines, acryloyls, arylating agents, vinyl sulfones, pyridyl disulfides, TNB-thiols and disulfide reducing agents. Most of these groups conjugate to sulfhydryls by either alkylation (usually the formation of a thioether bond) or disulfide exchange (formation of a disulfide bond). Such linkers further comprise a group, which is reactive to an amino group, like e.g. isothiocyanates, isocyanates, acyl azides, NHS (N-hydroxysuccinimide) esters, sulfonyl chlorides, aldehydes, glyoxals, epoxides, oxiranes, carbonates, aryl halides, imidoesters, carbodiimides, anhydrides, and fluorophenyl esters. Most of these conjugate to amines by either acylation or alkylation. An example for such a linker is SMCC (succinimidyl 4-(N-maleimidomethyl)cyclohexane-1-carboxylate). A preferred non-protein polymer is Glucosamine (GlcN) coupled to SMCC, i.e. SMCC-GlcN.

If the IL-4 muteins are expressed in a prokaryotic host cell, e.g. in *E. coli,* the IL-4 mutein may be expressed into inclusion bodies. While refolding the IL-4 mutein in an oxidative environment, glutathione may be added in a redox couple. If the IL-4 mutein is not modified thereafter, the non-protein polymer is glutathione. Accordingly, the present invention relates to an embodiment, wherein the non-protein polymer is glutathione.

Fig. 10 shows an overview of preferred amino acid sequences of the IL-4 muteins of the invention. The IL-4 mutein may comprise a sequence as depicted in any of SEQ ID NOs: 2-10. The IL-4 mutein may essentially consist of a sequence as depicted in any of SEQ ID NOs: 2-10. The IL-4 mutein may consist of a sequence as depicted in any of SEQ ID NOs: 2-10. The IL-4 mutein may also comprise an amino acid sequence with at least 75 %, at least 80 %, at least 85 %, at least 90 % at least 95 % or at least 99 % sequence identity to an amino an amino acid sequence selected from the group consisting of SEQ ID NOs: 2-10, wherein the IL-4 mutein comprises at least one amino acid, which allows site-directed conjugation of a non-protein polymer and wherein a non-protein polymer is conjugated to the IL-4 mutein via the amino acid, which allows site-directed conjugation of a non-protein polymer. Preferably, the IL-4 mutein either comprises the mutation K117C or the mutation R121C, more preferably the IL-4 mutein either comprises the mutation K117C or the mutation R121C, and in addition the mutation F82D.

The IL-4 mutein may also essentially consist of an amino acid sequence with at least 75 %, at least 80 %, at least 85 %, at least 90 % at least 95 % or at least 99 % sequence identity to an amino an amino acid sequence selected from the group consisting of SEQ ID NOs: 2-10, wherein the IL-4 mutein comprises at least one amino acid, which allows site-directed conjugation of a non-protein polymer and wherein a non-protein polymer is conjugated to the IL-4 mutein via the amino acid, which allows site-directed conjugation of a non-protein polymer. Preferably, the IL-4 mutein either comprises the mutation K117C or the mutation R121C, more preferably the IL-4 mutein either comprises the mutation K117C or the mutation R121C, and in addition the mutation F82D.

The IL-4 mutein may also consist of an amino acid sequence with an amino acid sequence selected from the group consisting of SEQ ID NOs: 2-10, wherein the IL-4 mutein comprises at least one amino acid, which allows site-directed conjugation of a non-protein polymer and wherein a non-protein polymer is conjugated to the IL-4 mutein via the amino acid, which allows site-directed conjugation of a non-protein polymer.

The term "homology" as used herein in its usual meaning and includes identical amino acids as well as amino acids which are regarded to be conservative substitutions (for example, exchange of a glutamate residue by an aspartate residue) at equivalent positions in the linear amino acid sequence of two proteins that are compared with each other. By "identity" or "sequence identity" is meant a property of sequences that measures their similarity or relationship. The term "sequence identity" or "identity" as used in the present invention means the percentage of pair-wise identical residues - following (homology) alignment of a sequence of a polypeptide of the invention with a sequence in question - with respect to the number of residues in the longer of these two sequences. Identity is measured by dividing the number of identical residues by the total number of residues and multiplying the product by 100.

The percentage of sequence homology or sequence identity can, for example, be determined herein using the program BLASTP, version blastp 2.2.5 (November 16, 2002; cf. Altschul, S. F. et al. (1997) Nucl. Acids Res. 25, 3389-3402). In this embodiment the percentage of homology is based on the alignment of the entire polypeptide sequences (matrix: BLOSUM 62; gap costs: 11.1; cutoff value set to 10⁻³) optionally including the propeptide sequences, using the human IL-4 as reference in a pairwise comparison. It is calculated as the percentage of numbers of "positives" (homologous amino acids) indicated as result in the BLASTP program output divided by the total number of amino acids selected by the program for the alignment. It is noted in this connection that this total number of selected amino acids can differ from the length of the human IL-4.

The present invention also relates to a nucleic acid encoding the IL-4 mutein. Such a nucleic acid may be comprised in a vector. Preferably, the vector further comprises a promoter operatively linked to the nucleic acid. Such a promoter is suitable to induce transcription of a gene and thereby induces the expression of the nucleic acid.

Promoters are untranscribed sequences located upstream (i.e., 5') to the start codon of a structural gene (generally within about 100 to 1000 bp) that control the transcription of the structural gene. Promoters are conventionally grouped into one of two classes: inducible promoters and constitutive promoters. Inducible promoters initiate increased levels of transcription from DNA under their control in response to some change in culture conditions, such as the presence or absence of a nutrient or a change in temperature. Constitutive promoters, on the other hand, initiate continual gene product production; that is, there is little or no control over gene expression. A large number of promoters, recognized by a variety of potential host cells, are well known. A heterologous promoter is preferred, however, if it permits greater transcription and higher yields of the expressed protein as compared to the native promoter, and if it is compatible with the host cell system that has been selected for use.

Promoters suitable for use with prokaryotic hosts include the beta-lactamase and lactose promoter systems; alkaline phosphatase; a tryptophan (trp) promoter system; and hybrid promoters such as the tac promoter. Other known bacterial promoters are also suitable. Their sequences have been published, thereby enabling one skilled in the art to ligate them to the desired DNA sequence, using linkers or adapters as needed to supply any useful restriction sites.

Suitable promoters for use with yeast hosts are also well known in the art. Yeast enhancers are advantageously used with yeast promoters. Suitable promoters for use with mammalian host cells are well known and include, but are not limited to, those obtained from the genomes of viruses such as polyoma virus, fowl pox virus, adenovirus (such as Adenovirus 2), bovine papilloma virus, avian sarcoma virus, cytomegalovirus, retroviruses, hepatitis-B virus and most preferably Simian Virus 40 (SV40). Other suitable mammalian promoters include heterologous mammalian promoters, for example, heat-shock promoters and the actin promoter.

The present invention further relates to a host cell comprising the nucleic acid or the vector of the invention. Suitable host cells may be prokaryotic or eukaryotic. A preferred prokaryotic host cell is *E. coli.* Eukaryotic cells are able to recognize glycosylation signals and conjugate glycans to the asparagine of the signal sequence to form N-glycosylated proteins. Accordingly, the host cell preferably is eukaryotic. Eukaryotic host cells may include yeast, like e.g. *Saccharomyces, Pichia pastoris, Kluyveromyces lactis, Hansenula polymorpha, Yarrowia lipolytica.* Other preferred host cells are Sf9 cells. Preferably, the host cell is mammalian. Suitable mammalian host cells are for example CHO cells, HEK293 cells, Expi293 cells, HeLa cells, U2OS cells, A549 cells, HT1090 cells, CAD cells, P19 cells, NIH 3T3 cells, L020 cells, N2a cells, MCF-7 cells, Y79 cells, SO-Rb50 cells, HepG2 cells, DUKX-X11 cells, J558L cells and BHK cells.

The present invention further relates to a method for producing an IL-4 mutein according to the invention, comprising the steps of: culturing the host cell under conditions allowing the production of the IL-4 mutein and obtaining the IL-4 mutein.

"Conditions allowing the production of the IL-4 mutein" as used herein relate at least to the choice of a suitable culture medium, a suitable temperature or a suitable container or incubator. Different host cells need different culture media. Examples for the conditions allowing the production of the IL-4 mutein are shown in the Examples (see materials and methods). A person skilled in the art is capable of choosing the culture conditions to ensure sufficient and efficient protein expression.

"Obtaining the IL-4 mutein" as used herein relates to the isolation or purification of the IL-4 mutein, i.e. the separation of the IL-4 mutein from the other parts of the host cells. Possible steps include the lysis of the host cell and a further purification. In one example, the IL-4 muteins expressed in *E. coli are* expressed in inclusion bodies. A person skilled in the art knows how to separate inclusion bodies. Another way to isolate the IL-4 mutein could be the use of affinity chromatography. For exemplary means and methods to obtain the IL-4 mutein see the materials and methods section of the Examples.

A further optional step includes the conjugation of the IL-4 mutein with a non-protein polymer, preferably as described herein. However, as not all IL-4 mutein may be successfully conjugated, the method may further comprise the step removing non-conjugated IL-4 muteins, which is exemplified in the Examples.

The method may comprise further a step of enzymatic elongation of chemically coupled mono- and/or oligosaccharides. The enzymatic elongation of chemically coupled mono- and/or disaccharides may be done by employing glycosyltransferases and/or other glycan-processing enzymes. Examples for such enzymatic elongation is disclosed in Gamblin et al. (2004), Angew Chem Int Ed, 43:828-833

The IL-4 mutein may be comprised in a pharmaceutical composition. The pharmaceutical composition of the present invention may be suitable or adapted for inhalation or subcutaneous injection. For injectable formulations, the pharmaceutical compositions can be in lyophilized powder in admixture with suitable excipients in a suitable vial or tube. Before use in the clinic, the drugs may be reconstituted by dissolving the lyophilized powder in a suitable solvent system for form a composition suitable for intravenous or intramuscular injection.

Pharmaceutical compositions provided herein may be provided in a sterile injectable form, i.e. in a form that is adapted for subcutaneous injection or intravenous infusion. For example, in some embodiments, pharmaceutical compositions are provided in a liquid dosage form that is suitable for injection. In some embodiments, pharmaceutical compositions are provided as powders (*e.g.* lyophilized and/or sterilized), optionally under vacuum, which are reconstituted with an aqueous diluent (e.g., water, buffer, salt solution, etc.) prior to injection. In some embodiments, pharmaceutical compositions are diluted and/or reconstituted in water, sodium chloride solution, sodium acetate solution, benzyl alcohol solution, phosphate buffered saline, etc. In some embodiments, powder should be mixed gently with the aqueous diluent (e.g., not shaken).

Pharmaceutical compositions provided herein may also be inhalable. Such an inhalable IL-4 and IL-13 mutein pharmaceutical compositions are, for example, disclosed in WO 2009/009775.

The pharmaceutical compositions contemplated as part of the present invention are not limited to pharmaceutical compositions that are injectable or inhalable, but include all types of pharmaceutical compositions commonly known and used in the art.

The pharmaceutical compositions may further comprise one or more pharmaceutically acceptable excipients. In some embodiments, pharmaceutical compositions comprise one or more preservatives. In some embodiments, pharmaceutical compositions comprise no preservative. Excipients as used herein may be or comprise solvents, dispersion media, diluents, or other liquid vehicles, dispersion or suspension aids, surface active agents, isotonic agents, thickening or emulsifying agents, preservatives, and the like, as suited to the particular dosage form desired. Remington's The Science and Practice of Pharmacy, 21st Edition, A. R. Gennaro, (Lippincott, Williams & Wilkins, Baltimore, MD, 2006) discloses a variety of excipients used in formulating pharmaceutical compositions and known techniques for the preparation thereof. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The compositions may contain pharmaceutically acceptable auxiliary substances as required. Acceptable auxiliary substances preferably are nontoxic to recipients at the dosages and concentrations employed. The pharmaceutical composition can contain auxiliary substances for modifying, maintaining, or preserving, for example, the pH, osmolality, viscosity, clarity, color, isotonicity, odor, sterility, stability, rate of dissolution or release, adsorption, or penetration of the composition. Suitable formulation materials include, but are not limited to, amino acids (such as glycine, glutamine, asparagine, arginine, or lysine), antimicrobials, antioxidants (such as ascorbic acid, sodium sulfite, or sodium hydrogen- sulfite), buffers (such as borate, bicarbonate, Tris-HCI, citrates, phosphates, or other organic acids), bulking agents (such as mannitol or glycine), chelating agents (such as ethylenediamine tetraacetic acid (EDTA)), complexing agents (such as caffeine, polyvinylpyrrolidone, beta-cyclodextrin, or hydroxypropyl-beta-cyclodextrin), fillers, monosaccharides, disaccharides, and other carbohydrates (such as glucose, mannose, or dextrins), proteins (such as serum albumin, gelatin, or immunoglobulins), coloring, flavoring and diluting agents, emulsifying agents, hydrophilic polymers (such as polyvinylpyrrolidone), low molecular weight polypeptides, salt-forming counterions (such as sodium), preservatives (such as benzalkonium chloride, benzoic acid, salicylic acid, thimerosal, phenethyl alcohol, methyl paraben, propyl paraben, chlorhexidine, sorbic acid, or hydrogen peroxide), solvents (such as glycerin, propylene glycol, or polyethylene glycol), sugar alcohols (such as mannitol or sorbitol), suspending agents, surfactants or wetting agents (such as pluronics; PEG; sorbitan esters; polysorbates such as polysorbate 20 or polysorbate 80; triton; tromethamine; lecithin; cholesterol or tyloxapal), stability enhancing agents (such as sucrose or sorbitol), tonicity enhancing agents (such as alkali metal halides - preferably sodium or potassium chloride - or mannitol sorbitol), delivery vehicles, diluents, excipients and/or pharmaceutical adjuvants. See Remington's Pharmaceutical Sciences (18th Ed., A.R. Gennaro, ed., Mack Publishing Company 1990.

A pharmaceutical composition in accordance with the invention may be prepared, packaged, and/or sold in bulk, as a single unit dose, and/or as a plurality of single unit doses. As used herein, a "unit dose" is discrete amount of the pharmaceutical composition comprising a predetermined amount of the active ingredient.

Relative amounts of active ingredient, pharmaceutically acceptable excipient, and/or any additional ingredients in a pharmaceutical composition in accordance with the invention may vary, depending upon the identity, size, and/or condition of the subject treated and/or depending upon the route by which the composition is to be administered. By way of example, the composition may comprise between 0.1% and 100% (w/w) active ingredient, i.e. the IL-4 mutein. The optimal pharmaceutical composition can be determined by a skilled artisan depending upon, for example, the intended route of administration, delivery format, and desired dosage. See, e.g., Remington's Pharmaceutical Sciences, supra.

The present invention also relates to a pharmaceutical kit of parts comprising the pharmaceutical composition of the invention and an anti-inflammatory agent. Examples for an anti-inflammatory agent include, but are not limited to, corticosteroids such as hydrocortisone, methylprednisolone, prednisolone, prednisone, triamcinolone, Amcinonide, budesonide, desonide, fluocinolone acetonide, fluocinonide, halcinonide, and triamcinolone acetonide, Beclometasone, betamethasone, dexamethasone, fluocortolone, halometasone, mometasone Alclometasone dipropionate, betamethasone dipropionate, betamethasone valerate, clobetasol propionate, clobetasone butyrate, fluprednidene acetate, mometasone furoate Ciclesonide, cortisone acetate, hydrocortisone aceponate, hydrocortisone acetate, hydrocortisone buteprate, hydrocortisone butyrate, hydrocortisone valerate, prednicarbate, or tixocortol pivalate; or nonsteroidal anti-inflammatory drugs such as Salicylates (Aspirin (acetylsalicylic acid), Diflunisal (Dolobid), Salicylic acid and other salicylates, Salsalate (Disalcid), Propionic acid derivatives (Ibuprofen, Dexibuprofen, Naproxen, Fenoprofen, Ketoprofen, Dexketoprofen, Flurbiprofen, Oxaprozin, Loxoprofen), acetic acid derivatives (Indomethacin, Tolmetin, Sulindac, Etodolac, Ketorolac, Diclofenac, Aceclofenac, Nabumetone), Enolic acid (Oxicam) derivatives (Piroxicam, Meloxicam, Tenoxicam, Droxicam, Lornoxicam, Isoxicam, Phenylbutazone (Bute)), Anthranilic acid derivatives (Fenamates: Mefenamic acid, Meclofenamic acid, Flufenamic acid, Tolfenamic acid), Selective COX-2 inhibitors (Coxibs: Parecoxib, Lumiracoxib, Etoricoxib, Firocoxib) Sulfonanilides, Nimesulide, Clonixin, Licofelone, or H-harpagide.

The IL-4 mutein of the invention, the pharmaceutical composition of the invention and the pharmaceutical kit of parts of the invention are useful in treating a subject. A subject that may profit from such a treatment may suffer from an inflammatory disease. As outlined above, IL-4 and IL-13 induce upregulation of vascular cell adhesion molecule-1 (VCAM1) on endothelial cells and thus facilitate leucocyte migration from the blood to the site of infection. Further, IL-4 and IL-13 upregulate Fcε-receptors for IgE immunoglobulins on mast cells, eosinophils and B-cells, thereby enhancing IgE-dependent responses. IL-4/IL-13-mediated IgE sensitization is of great pathophysiological significance, as IgE antibodies play a major role in the onset and progression of various atopic diseases, such as asthma, atopic dermatitis and allergic rhinitis. The recognition of a specific antigen/allergen through the IgE/Fcε complex on mast cells as well as basophilic and eosinophilic granulocytes stimulates degranulation of the cell leading to the release of pro-inflammatory mediators, like the anticoagulant heparin and the vasodilator histamine, which promote transport of immune cells to the site of infection. In addition, prostaglandins, leukotrienes and proteases released upon degranulation facilitate local inflammation by causing muscle constriction and tissue remodeling. Accordingly, the IL-4 mutein of the invention, the pharmaceutical composition of the invention and the pharmaceutical kit of parts of the invention are useful in treating a subject with an inflammatory disease characterized by the actions of IL-4 and/or IL-13.

Accordingly, the present invention relates to the IL-4 mutein of the invention, the pharmaceutical composition of the invention or the pharmaceutical kit of parts of the invention for use in a method of treatment. Preferably, the IL-4 mutein of the invention, the pharmaceutical composition of the invention or the pharmaceutical kit of parts of the invention are for use in a method of treating an inflammatory disease. The inflammatory disease preferably is asthma, arthritis, colitis ulcera, atopic dermatitis, allergy and allergic asthma. In another preferred embodiment, the IL-4 mutein of the invention, the pharmaceutical composition of the invention or the pharmaceutical kit of parts of the invention are for use in a method of treating leishmaniosis. The subject preferably is human. A person skilled in the art is able to determine the correct dosage of the IL-4 mutein of the invention for use in methods of treatment.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "less than" or in turn "more than" does not include the concrete number.

For example, less than 20 means less than the number indicated. Similarly, more than or greater than means more than or greater than the indicated number, e.g. more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications cited throughout the text of this specification (including all patents, patent application, scientific publications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The content of all documents and patent documents cited herein is incorporated by reference in their entirety.

An even better understanding of the present invention and of its advantages will be evident from the following examples, offered for illustrative purposes only. The examples are not intended to limit the scope of the present invention in any way.

The invention is also characterized by the following items:
Item 1: A IL-4 mutein capable of inhibiting IL-4 and/or IL-13 signalling, wherein the IL-4 mutein comprises an amino acid mutation at each of sequence positions 82 and 121 in comparison to the human mature IL-4 (positions 25-153 of UniProt database entry P05112, SEQ ID NO: 1), wherein the amino acid at sequence position 82 is mutated to aspartic acid and the amino acid at sequence position 121 is mutated to an amino acid, which allows site-directed conjugation of a non-protein-polymer, and wherein a non-protein-polymer is conjugated to the mutein via the mutated amino acid at sequence position 121.
Item 2: An IL-4 mutein capable of inhibiting IL-4 and/or IL-13 signalling, wherein the IL-4 mutein comprises an amino acid mutation at each of sequence positions 82 and 117 in comparison to the human mature IL-4 (positions 25-153 of UniProt database entry P05112, SEQ ID NO: 1), wherein the amino acid at position 82 is mutated to aspartic acid and the amino acid at position 117 is mutated to an amino acid, which allows site-directed conjugation of a non-protein-polymer, and wherein a non-protein-polymer is conjugated to the IL-4 mutein via the mutated amino acid of position 117.
Item 3: The mutein of item 2, wherein the mutein is an antagonist of IL-4 receptor type II.
Item 4: The IL-4 mutein of item 2, wherein the IL-4 mutein comprises a further mutation at position 124, wherein the amino acid at position 124 is mutated to an amino acid selected from the group consisting of glycine, lysine and asparagine.
Item 5: The IL-4 mutein of any of the preceding items, further comprising at least one mutated amino acid at a sequence position selected from the group consisting of sequence positions 20, 28 and 61.
Item 6: The IL-4 mutein of items 1 to 4, further comprising two mutated amino acids at sequence positions selected from the group consisting of sequence positions 20, 28 and 61.
Item 7: The IL-4 mutein of items 1 to 4, further comprising three mutated amino acids at sequence positions selected from the group consisting of sequence positions 20, 28 and 61.
Item 8: The IL-4 mutein of items 5-7, wherein the at least one, two or three mutated amino acid(s) at a sequence position selected from the group consisting of sequence positions 20, 28 and 61 provides one additional glycosylation site at each mutated sequence position.
Item 9: Item The IL-4 mutein of items 5 to 8, wherein the IL-4 mutein comprises asparagine at each mutated sequence position selected from the group consisting of sequence positions 20, 28 and 61.
Item 10: The IL-4 mutein of items 5 to 9, wherein the IL-4 mutein is N-glycosylated at at least one sequence position selected from the group consisting of sequence positions 20, 28 and 61.
Item 11: The IL-4 mutein of items 5 to 7, wherein the at least one, two or three mutated amino acid(s) at a sequence position selected from the group consisting of sequence positions 20, 28 and 61 is mutated to an amino acid, which allows site-direct conjugation of a non-protein polymer and wherein a non-protein polymer is conjugated to the IL-4 mutein via the mutated amino acid at a sequence position selected from the group consisting of positions 20, 28 and 61.
Item 12: The IL-4 mutein of any of the preceding items, further comprising a mutated amino acid at sequence position 38, wherein the amino acid at sequence position 38 is mutated to an amino acid, which allows site-directed conjugation of a non-protein-polymer and wherein a non-protein polymer is conjugated to the IL-4 mutein via the mutated amino acid of sequence position 38.
Item 13: The IL-4 mutein of any of the preceding items, comprising an amino acid sequence with at least 75 %, at least 80 %, at least 85 %, at least 90 %, at least 95 % or at least 99 % sequence identity to an amino acid sequence selected from the group consisting of SEQ ID NOs: 2-10, wherein the IL-4 mutein comprises at least one amino acid, which allows site-directed conjugation of a non-protein polymer and wherein a non-protein polymer is conjugated to the IL-4 mutein via the amino acid, which allows site-directed conjugation of a non-protein polymer.
Item 14: The IL-4 mutein of any of the preceding items, consisting of an amino acid sequence with at least 75 %, at least 80 %, at least 85 %, at least 90 %, at least 95 % or at least 99 % sequence identity to a sequence selected from the group consisting of SEQ ID NOs: 2-10, wherein the IL-4 mutein comprises at least one amino acid, which allows site-directed conjugation of a non-protein polymer and wherein a non-protein polymer is conjugated to the IL-4 mutein via the amino acid, which allows site-directed conjugation of a non-protein polymer.
Item 15: The IL-4 mutein of any of the preceding items that has an amino acid sequence selected from the group consisting of SEQ ID NOs: 2-10, wherein the IL-4 mutein comprises at least one amino acid, which allows site-directed conjugation of a non-protein polymer and wherein a non-protein polymer is conjugated to the IL-4 mutein via the amino acid, which allows site-directed conjugation of a non-protein polymer..
Item 16: The IL-4 mutein of any of the preceding items, wherein the amino acid, which allows site-directed conjugation of a non-protein-polymer, is cysteine.
Item 17: The IL-4 mutein of any of the preceding items, wherein the non-protein polymer comprises a mono- or oligosaccharide.
Item 18: The IL-4 mutein of any of the preceding items, wherein the non-protein polymer comprises polysialic acid or a complex N-glycan-like oligosaccharide structure.
Item 19: The IL-4 mutein of item 17 or 18, wherein the non-protein polymer is chemically modified to allow conjugation to an amino acid, which allows site-directed conjugation of a non-protein polymer.
Item 20: The IL-4 mutein of item 19, wherein the non-protein polymer comprises a thiol group or is coupled to SMCC (succinimidyl 4-(N-maleimidomethyl)cyclohexane-1-carboxylate).
Item 21: The IL-4 mutein of any of the preceding items, wherein the IL-4 mutein has a glycosylation pattern obtained by an expression in a eukaryotic cell.
Item 22: The IL-4 mutein of item 21, wherein the glycosylation pattern is obtained by expression in a mammalian cell.
Item 23: A nucleic acid encoding the IL-4 mutein of any one of items 1-22.
Item 24: A vector comprising the nucleic acid of item 23.
Item 25: The vector of item 24, further comprising a promoter operatively linked to the nucleic acid.
Item 26: A host cell comprising the nucleic acid of item 23 or the vector of item 24 or 25.
Item 27: The host cell of item 26, wherein the host cell is eukaryotic, preferably mammalian.
Item 28: A method for producing an IL-4 mutein according to any of items 1 to 22, comprising the steps of:
   (a) culturing the host cell of item 23 or 24 under conditions allowing the production of the IL-4 mutein,
   (b) obtaining the IL-4 mutein,
   (c) optionally conjugating the IL-4 mutein with a non-protein polymer,
   (d) optionally removing non-conjugated IL-4 muteins, and
   (e) optionally enzymatic elongation of chemically coupled mono- and/or oligosaccharides.
Item 29: A pharmaceutical composition comprising the IL-4 mutein of any one of items 1-22.
Item 30: The pharmaceutical composition of item 29, wherein the pharmaceutical composition is adapted for inhalation.
Item 31: The pharmaceutical composition of item 29, wherein the pharmaceutical composition is adapted for subcutaneous injection.
Item 32: A pharmaceutical kit of parts comprising the pharmaceutical composition of item 29 and an anti-inflammatory agent.
Item 33: The IL-4 mutein of any of items 1-22, the pharmaceutical composition of any of items 29-31 or the pharmaceutical kit of parts of item 32 for use in a method of treating a subject.
Item 34: The IL-4 mutein of any of items 1-22, the pharmaceutical composition of any of items 29-31 or the pharmaceutical kit of parts of item 32 for use in a method of treating an inflammatory disease.
Item 35: The IL-4 mutein of any of items 1-22, the pharmaceutical composition of any of items 29-31 or the pharmaceutical kit of parts of item 32 for use of item 34, wherein the inflammatory disease is selected from the list consisting of asthma, arthritis, colitis ulcera, atopic dermatitis, allergy and allergic asthma.
Item 36: The IL-4 mutein of any of items 1-22, the pharmaceutical composition of any of items 29-31 or the pharmaceutical kit of parts of item 32 for use in a method of treating leishmaniosis.
Item 37: The IL-4 mutein for use of any one of items 33-36, wherein the subject is human.

### EXAMPLES

### Materials and methods

### Chemicals

*E. coli* glutaredoxin (GRX-01) was obtained from Cayman Chemical, glutathione and NADPH was obtained from Carl Roth GmbH (Karlsruhe, Germany), and yeast glutathione reductase (GR) was from Sigma-Aldrich (St. Louis, MO, USA). Succinimidyl 4-(N-maleimidomethyl) cyclohexane-1-carboxylate (SMCC) was purchased from Apollo Scientific (Cheshire, UK) and D(+) glucosamine hydrochloride was obtained from Merck (Darmstadt, Germany). Phenylselenylbromide, 1-thio-β-D-glucose sodium salt and 1-thio-β-D-glucose tetraacetate was ordered from Sigma-Aldrich (St. Louis, USA). Sequencing grade trypsin was acquired from Promega (Fitchburg, USA).

### Production and reduction of IL4 variants in E. coli for site-directed chemical glycosylation

The cDNA-encoding IL-4 cysteine muteins were synthesized by two-step mutagenesis PCR. Expression, purification and enzymatic reduction of *E. coli* derived IL-4 cysteine variants were essentially performed as described by Duppatla et al. 2012. The following primers were used for mutagenesis:

**Table 1: List of primers used for mutagenesis. (s: sense, as: anti-sense)**

| Name | Sequence | SEQ ID NO |
|---|---|---|
| R121D Y124D_s | GACGATCATGGACGAGAAAGATTC | 11 |
| R121D Y124D_as | GAATCTTTCTCGTCCATGATCGTC | 12 |
| F82D_s | CAGCTGATCCGAGACCTGAAACGG | 13 |
| F82D_as | CGTTTCAGGTCTCGGA TCAGCTG | 14 |
| N38C_s | CTGCCTCCAAGTGCACAACTGAGAAG | 15 |
| N38C_as | CTTCTCAGTTGTGCACTTGGAGGCAG | 16 |
| R121C_s | GGCTAAAGACGATCATGTGTGAGAAATATTCAAAGTG | 17 |
| R121C_as | CACTTTGAATATTTCTCACACATGATCGTCTTTAGCC | 18 |
| Q20N_s | GCCTCACAGAGAACAAGACTCTGTGCACCG | 19 |
| Q20N_as | CGGTGCACAGAGTCTTGTTCTCTGTGAGGC | 20 |
| T28N_s | GCACCGAGTTGAACGTAACAGAC | 21 |
| T28N_as | GTCTGTTACGTTCAACTCGGTGC | 22 |
| K61N_s | CCACCATGAGAACGACACTCGCTGC | 23 |
| K61N_as | GCAGCGAGTGTCGTTCTCATGGTGG | 24 |
| K117N_T118A_ I119T_s | GGAAAGGCTAAACGCGACCATGAGAGAGAAATATTC | 25 |
| K117N_T118A_ I119T_as | GAATATTTCTCTCTCATGGTCGCGTTTAGCCTTTCC | 26 |
| R121N_K123S_s | CTAAAGACGATCATGAACGAGAGCTATTCAAAGTGTTC | 27 |
| R121N_K123S_a s | GAACACTTTGAATAGCTCTCGTTCATGATCGTCTTAG | 28 |
| N38Q_s | CTGCCTCCAAGCAGACAACTGAGAAG | 29 |
| N38Q_as | CTTCTCAGTTGTCTGCTTGGAGGCAG | 30 |

### Coupling to amine-containing monosaccharides (or oligosaccharides) via the bifunctional cross-linker SMCC

The cross-linker SMCC (succinimidyl 4-(N-maleimido-methyl)-cyclohexane-1-carboxylate) was dissolved in acetonitrile at a concentration of 100mM. A 400mM glucosamine solution was prepared in 100mM sodium phosphate, 4mM EDTA pH 8.0. Both solutions were mixed in 1:1 (v/v) ratio to yield a stoichiometric ratio (SMCC:glucosamine) of 1:4 and the mixture was incubated at 21°C for 2 h. The reaction was stopped by adding TFA to a final concentration of 0.1% (v/v). The SMCC-glucosamine coupling product was purified by subsequent RP-HPLC employing a C18 column (µRPC C2/C18 ST 4.6/100, GE Healthcare) and elution was monitored at 302nm (absorbance maximum of the maleimide group in SMCC). The SMCC-glucosamine conjugate eluted at 55-60% acetonitrile. Conjugate-containing fractions were pooled, analyzed by mass spectrometry and freeze-dried. For coupling to IL-4, thiol-activated IL-4 mutein was dissolved in 100 mM sodium phosphate pH 6.5, glucosamine-SMCC conjugate was dissolved acetonitrile to give a 5 mM concentration and both solutions were mixed in a molar ratio (protein:gluco-SMCC conjugate) of 1:100. After incubation for 2h at 21°C the reaction mixture was subsequently purified by RP-HPLC employing a C4 column (Jupiter C4, 10 µm, 250 × 10 mm, Phenomenex, Germany) and a gradient 0.1 % TFA to 100 % acetonitrile.

### Coupling of thiol-carbohydrates using phenylselenylbromide activation

Glyco-SeS reaction was performed as in van Kasteren et al. 2007. Activated IL-4 mutein was dissolved in buffer containing 10 mM CHES, 70mM MES, 2mM CaCl₂, pH 9.5 to a final concentration of 66 µM. Phenylselenylbromide was dissolved in acetonitrile at a concentration of 100 mM. The reaction was started by adding 40 equiv. (3 mM) of phenylselenyl bromide to the protein solution. The mixture was vortexed for 30 seconds and then placed on a shaker for 1 h at 21 °C. Non-reacted phenylselenylbromide and byproducts were removed by size exclusion chromatography using a PD10 column (GE Healthcare). Protein-containing fractions were pooled and stored at -20°C until further use. Thiol-group containing carbohydrates were dissolved in water at 30 mM concentration. A 66 µM protein-phenylselenyl conjugate was incubated with 3 mM of the above-described thiol-carbohydrate solution for 20min at 21 °C, to increase coupling yield further thiol-carbohydrate solution was added to raise the final concentration to 6 mM. The mixture was incubated again for 20 min at 21 °C. The IL4-carbohydrate conjugate was subsequently purified by RP-HPLC using a C4 column (Jupiter C4, 10 µm, 250 × 10 mm, Phenomenex, Germany) and employing a gradient 0.1% TFA to 100% acetonitrile.

### Direct coupling of thiol-carbohydrates during refolding

IL-4 protein either in form of E. *coli*-derived IL-4 inclusion bodies or purified from Hek293 cells was first denatured using 5.3 M GuHCl, 100 mM Tris pH 8.0, 1 mM DTT (conc. of 50 mg/mL for inclusion bodies or 1 mg/mL for Hek293T protein). After stirring for 2h at RT the clarified solution was added dropwise to 25-fold ice cold refolding buffer (1 M Arginine, 50 mM Tris HCI pH 8.0, 5 mM EDTA, 1 mM thiol-containing carbohydrate as non-protein polymer) while stirring and incubated at 4 °C for at least 72 h. His-tagged Hek293 cell derived IL-4 mutein was isolated using IMAC as stated above and subsequent dialysis against 1 mM HCI. E. *coli*-derived IL-4 mutein was purified using ion exchange chromatography (HiTrap CM Sepharose FF, GE Healthcare) and subsequent RP-HPLC using a C4 column. Protein containing fractions were freeze-dried for storage at -20°C.

### Removal of non-conjugated IL4 protein with iodoacetyl activated agarose beads (SulfoLink)

lodoacetyl-activated agarose beads (SulfoLink™, Thermo Scientific) were used to remove unreacted protein with free sulfhydryl groups after chemical glycosylation reaction. For the purification of 1 mg IL-4 protein 1 mL of the resin is added to 1 mg IL-4-conjugate mixture dissolved in 50mM Tris-HCI, 1mM EDTA pH 8.0. The suspension is mixed several times by inverting, for completion of the reaction the suspension is incubated at 21°C for 45min. The suspension is filled into a column jacket and the flow-through is collected. The protein solution is analyzed by SDS-PAGE and mass spectrometry, extensively dialyzed against 1 mM HCI and freeze-dried. If the protein pool still contains non-conjugated IL-4 mutein the purification step is repeated using fresh iodoacetyl-activated agarose.

### Cell experiments

HEKBlue IL-4/IL-13 cells (Invivogen) were cultured in a growth medium containing DMEM glutamax (Gibco), 10% heat inactivated FBS, 100 µg/mL streptomycin, 100 U/mL penicillin, 100 µg/mL zeocin, 10 µg/mL blasticidin at 37 °C and 5% CO2. Assays were performed in growth medium without phenol red. For the experiment cells were seeded in 96 well f-bottom plates (1x10⁴ cells/well). After 20-24 h of incubation with IL-4 the supernatant was taken and a PNPP assay was performed to assess SEAP induction. Absorbance was measured at 405/630 nm on a microplate reader.

TF-1 cells were grown in RMPI-1640 (Gibco) medium supplemented with 10% heat inactivated FBS, 100 µg/mL streptomycin, 100 U/mL penicillin and 8 ng/mL GM-CSF at 37 °C and 5% CO2. Proliferation assays were performed in RPMI-1640 with 10 % FBS, 100 µg/mL streptomycin, 100 U/mL penicillin without phenol red and GMCSF. Prior to administration of IL-4, cells were starved for four hours. For the experiment cells were seeded in 96 well f-bottom plates (4x10⁴ cells/well). After 72 h of incubation with IL-4, the proliferation rate was assessed by adding resazurin dye solution (0.15mg/ml in PBS) to the cells. After 4 h of incubation at 37°C to facilitate NADH/H⁺-dependent reduction to the red fluorescent resorufin, absorbance was measured at 571/749 nm on a microplate reader.

To evaluate the biologic activity of the IL-4 muteins cells were treated with 50 pM of IL-4 muteins. Signal intensity was normalized to 50 pM of wildtype IL-4. The Kᵢ-values of the IL-4 muteins were calculated from data obtained from dose-response curves. The half maximal effective concentration (EC₅₀) of wildtype IL-4 was determined by treating cells with a log 2 titration of IL-4 wildtype, starting at 10 nM concentration. To assess the half maximal inhibitory concentration (IC₅₀) of antagonistic IL-4 muteins, cells were treated with 50 pM of wildtype IL-4 in combination with a log 2 titration of IL-4 muteins, starting at 200 nM concentration. Signal intensity was normalized to 50 pM of wildtype IL-4.

### Proteolytic stability assay

Proteins were dissolved in 50 mM Tris-HCI, 1mM CaCl2 pH 7.6 and adjusted to a concentration of 30 - 32 µM. To about 500 µL of protein solution 10 µg of solubilized trypsin (Promega) was added. At different time points a sample was taken and mixed with 1:5 inhibitor cocktail (Merck). A SDS PAGE was performed with distinct amounts of sample and marker. After Coomassie staining gels were analysed with ImageLab software using the volume tool. Band intensities were normalized to a distinct marker band. Values were analysed statistically using the two-tailed t-test for independent samples (threshold for the declaration of statistical significance: P value < 0.05).

### Production biosynthetically glycosylated IL4 variants in Hek293 cells

IL4 cysteine muteins containing biosynthetic glycan structures as non-protein polymers were produced in transiently transfected HEK293 cells, namely high-expression cell lines such as Freestyle293™ or Expi293™ cells. Additional N-glycosylation sites were introduced by site-directed mutagenesis PCR (Primer sequence support) and cDNA was cloned into a modified version of the expression vector pHLsec (original pHLsec was provided by A. Radu Aricescu, University of Oxford) containing a C-terminal hexahistidine peptide tag and a downstream thrombin cleavage site, which allows subsequent removal of the His-tag if required.

Cells were grown in Expi293™ or Freestyle293™ expression medium (Invitrogen) accordingly supplemented with 100 U/ml penicillin and 100 µg/ml streptomycin in a humidified 8% CO₂ incubator at 37 °C and suspension (120 rpm). Prior to transfection, Expi293™ cells were adjusted to 2.5×10⁶ cells/mL and Freestyle293™ cells to 7.5×10⁵ cells/mL. Transfection was performed using PEI transfection reagent. Per ml of expression culture 1 µg vector-DNA was required. The DNA was added to OptiMEM medium (volume of OptiMEM is 3.5% (v/v) of the total culture volume) and mixed in a 1:1 ratio with OptiMEM (volume is 3% (v/v) of the total cell culture volume) supplemented with 2 µg/ml PEI (25 kDa linear, Polyscience). The transfection mixture was incubated at 21°C for 20 min and was then added dropwise to the cell culture. Five days after transfection, cells were centrifuged (6000 g, 15 min, 4 °C). The supernatant was dialysed twice against 10x volume of 5 mM NaH₂PO₄, 45 mM Na₂HPO₄, 300 mM NaCl and 10 mM Imidazole, pH 8.3. Prior to immobilized metal ion affinity chromatography (IMAC) (HisTrap Excel column, GE Healthcare), the protein solution was centrifuged again (20 min, 25000 g, 4 °C). Protein was eluted with the above-described buffer supplemented with 500 mM imidazole. IL-4-containing fractions are analyzed by SDS-PAGE, pooled, dialyzed against 1 mM HCI and freeze-dried.

### Example 1: Coupling of glycan structures to position 121 via a bifunctional chemical cross-linker

For chemical coupling a thiol group was introduced to generate a reactive site. A prokaryotic expression system has been chosen for production that produces IL-4 in insoluble form expressed in so-called inclusion bodies. After extraction, the non-folded protein requires an oxidative *in vitro* refolding to adopt its native four-helix bundle architecture stabilized by the three native disulphide bonds. Previous studies in our lab have shown that introducing the mutation F82D, i.e. exchanging Phe82 in the third helix by an aspartate, greatly increases refolding yield. In addition, this mutation augments the binding affinity of the IL-4 protein to its receptor IL-4Rα by a factor of about 5-fold (Kraich et al. 2006). To generate an IL-4 protein that can be converted to an antagonist, Arg121 was mutated in IL-4 F82D to a cysteine to yield the IL-4 mutein F82D R121C. The IL-4 mutein was expressed in *E. coli to* form insoluble inclusion bodies, where it was extracted from and solubilized in a buffer containing a chaotropic reagent. To facilitate proper formation of the three native disulphide bonds in IL-4 muteins, a glutathione-based redox couple was added during oxidative *in vitro* refolding. Under these conditions the unpaired cysteine residue introduced at position 121 (Cys121) formed a mixed disulphide with a glutathione moiety. While this is beneficial as it protects the thiol group of the unpaired cysteine from unwanted non-specific reactions and allows purification and long-term storage of the starting protein material, it also requires an additional step for the removal of this "protection" group before glycan coupling can proceed. Initial tests to cleave the mixed disulphide via chemical reduction were not successful, as all reducing agent tested resulted in opening of IL-4's native disulphide bonds, which led to denaturation and irreversible precipitation of the reduced protein.

Duppatla et al. 2012 developed a procedure employing an enzymatic removal of the glutathione moiety from the mixed protein-glutathione muteins using the E. *coli* enzyme glutaredoxin-1 (grxA). For this reaction a low concentration of glutathione in its reduced form (GSH) is used, which becomes oxidized and is again reduced in a catalytic cycle by glutathione reductase and NADPH. The low concentration of GSH allows to mainly reducing/deglutathionylating the mixed disulphide bond, but reaction times need to be optimized as longer incubation leads to reduction of also the native disulphide bonds of IL-4 with the same consequences as described above for chemical reduction. For IL-4 F82D R121C a reaction time of 2.5 min was sufficient to fully convert Cys121 into its unconjugated, free thiol form and protein could be recovered in high purity with about 80 % yield. Oxidation of the free thiol group was quenched by transferring the protein into buffer with pH lower than 6 which lead to protonation of the free thiol group and protects from oxidation processes.

First, complex glycans from natural protein sources were isolated. This might be achieved by enzymatic hydrolysis, e.g. cleavage via the endoglycosidase PNGase-F, which does not require harsh chemical conditions thereby minimizing the risk to chemically alter the glycan molecule. PNGase F hydrolyses the N-glycosidic bond between the asparagine residue of the protein and the first N-acetyl glucosamine (GlcNac) residue of the glycan chain, releasing a complex glycan with a single amino group at the anomeric carbon atom of the first GlcNac molecule. Coupling of this amino-sugar to Cys121 requires a (hetero)-bifunctional chemical cross-linker that has both a thiol-reactive and an amino-reactive group (Figure 2). Most importantly, the reaction can be performed under near physiological conditions. As bifunctional cross-linker succinimidyl 4-(N-maleimidomethyl) cyclohexane-1-carboxylate (SMCC) was chosen.

A study was performed by using the commercially available monosaccharide 2-glucosamine, which carries the amino function at C-atom 2 next to the anomeric C-atom. First, the glucosamine-SMCC conjugate was prepared to prevent unwanted random labeling of IL-4 primary amino residues, e.g. lysines, via NHS reaction. SMCC was coupled to 2-glucosamine in a molar ration of 1:4, using a solvent containing 50 % acetonitrile to increase the solubility of the hydrophobic SMCC. Unfortunately, mass spectrometry analysis of the coupling product showed a rather low conversion rate, making a purification of the SMCC-glucosamine adduct essential to avoid the presence of amino-reactive, non-glucosamine coupled SMCC in the subsequent coupling reaction with the IL-4 protein. A reversed-phase chromatography was chosen as the SMCC moiety exhibits rather high hydrophobicity, which is decreased when coupled to the quite hydrophilic glucosamine molecule. A µRPC C2/C18 ST 4.6/100 column was used to separate the coupling product from non-reacted educts. While the SMCC-glucosamine conjugate was eluted at 55 % acetonitrile concentration, the non-reacted SMCC required 90 % acetonitrile to be released form the reversed-phase resin. The overall yield for the conjugate was estimated to about 5 % only (based on SMCC as educt). Mass spectrometry confirmed the purity of the conjugate, which was then reacted in a molar ratio of 100:1 in an aqueous buffer system for 2 h at room temperature (RT) with enzymatic activated IL-4 F82D R121C. After purification of the mutein-SMCC-glucosamine conjugate (about 50 % yield), mass spectrometry confirmed that the mutein-glyco-conjugate can be indeed prepared and coupling to the mutein occurred specifically to the activated thiol group.

### Example 2: Linker-free coupling of thiol-carbohydrates using phenylselenylbromide activation

A different approach, which would allow direct conjugation of a glycan moiety to the engineered cysteine residue Cys121 and Cys117, was developed. A glyco-engineering strategy, established by Gamblin and colleagues that is termed Glyco-SeS, implements thiol-functionalized carbohydrates for site-directed linker-free conjugation to cysteine residues. Reaction with a phenylselenylating reagent converts the free sulfhydryl group of an engineered cysteine residue into the corresponding mixed phenylselenylsulphide (Bernardes et al. 2006, Gamblin et al. 2004, van Kasteren et al. 2007). Because of the resulting electrophilic nature of the sulphur atom in the S-Se bond, it readily reacts with a thiol-containing carbohydrate to form a disulphide-linked protein-glycan conjugate. This strategy can also be employed in an alternative setup, where the thio-carbohydrate is first activated with phenylselenylbromide, which can then be coupled to a free unpaired cysteine residue in the IL-4 mutein.

This approach has been shown to be applicable to glycans isolated from natural sources, although the glycan compound first has to be thiol-functionalized, e.g. through reaction with Lawesson's reagent (Bernardes et al. 2006). To demonstrate the feasibility of this approach for IL-4 mutein F82D R121C, the commercially available thiol-carbohydrates 1-thio-β-D-glucose and the acetylated derivative 1-thio-β-D-glucose tetraacetate were used. Enzymatically deglutathionylated and purified IL-4 mutein F82D R121C protein was treated with 40-fold molar excess of phenylselenyl bromide for 1 h at RT to yield the selenylsulphide intermediate (Figure 3). After desalting, the purified IL-4 mutein was then reacted with an 80-fold molar excess of the respective thio-glycan for 40 min at RT to provide the disulphide linked glycan-mutein conjugate. However, full conversion could not be achieved, mass spectrometry analysis revealed a minor contamination, which exhibited the molecular weight of unmodified IL-4 mutein F82D R121C. Because non-conjugated IL-4 mutein F82D R121C exerts agonistic activity, unmodified protein had to be removed. Since the agonistic contaminant carries the free thiol-group in its reduced and reactive state, non-conjugated IL-4 F82D R121C was removed by coupling to iodoacetyl-activated agarose thereby yielding homogenous glycosylated IL-4 F82D R121C-SS-(4ac)Glc and IL4 F82D R121C-SS-Glc, respectively. The results demonstrate that the Glyco-SeS method can be used for a cross-linker-free preparation of a disulphide-linked IL-4 glycoprotein.

### Example 3: Direct coupling of thiol-carbohydrates during refolding of bacterially derived IL-4

The oxidative refolding protocol for renaturation of IL-4 cysteine muteins from inclusion bodies utilizes a glutathione GSH/GSSG redox couple to facilitate the formation of native disulphide bonds. Reduced glutathione (GSH) thereby unspecifically cleaves disulphide bonds whereas oxidized glutathione (GSSG) induces formation of new disulphide bonds. Thus, a slow disulphide reshuffling occurs that facilitates formation and stabilization of a proteins native disulphide bonds and conformation. We wondered if a disulphide linked IL-4 glyco-conjugate could be generated in a similar way, by adding a thioglycan instead of glutathione to the refolding buffer, thereby rendering enzymatic liberation of the thiol group from a mixed disulfide glutathione conjugate void (Figure 4).

Bacterially derived inclusion bodies harboring IL-4 mutein F82D R121C were solubilized using a buffer containing guanidine hydrochloride and DTT. After incubation for 2 h at RT, the protein solution was added to 25 volumes ice-cold renaturation buffer to yield a protein concentration of about 50 µg/ml. The renaturation buffer contained 1 M arginine to reduce protein aggregation. Instead of supplementing the buffer with a glutathione redox couple (5 mM oxidized glutathione, 2 mM reduced glutathione) 1 mM 1-β-thio-glucose (tetraacetate) as non-protein polymer was added. Surprisingly, even though no oxidizing redox partner such as oxidized glutathione was present, the refolding yield was identical to that obtained for refolding buffer comprising the complete glutathione redox couple (about 10 mg protein per gram *E. coli* cell biomass). Mass spectrometry verified that all three native disulphide bonds were formed and that Cys121 was completely disulphide bonded to glucose (tetraacetate) with no non-conjugated species being present.

### Example 4: Glycosylated IL-4 F82D R121C muteins display increased IL-4Rα receptor binding

To test whether the coupling of thio-glycans affects the receptor binding properties of the IL-4 mutein to the high-affinity receptor IL-4Rα, which could deteriorate the antagonistic efficacy of the IL-4 glycan-conjugates, *in vitro* interaction analysis employing surface plasmon resonance (SPR) was used to determine the binding kinetics and affinities of the glyco-engineered IL-4 F82D R121C muteins towards the IL-4Rα receptor. The ectodomain of IL-4Rα was immobilized onto a streptavidin-coated biosensor chip using site-specifically biotinylated IL-4Rα ectodomain protein. This biosensor was then perfused with IL-4 mutein-glycan-conjugates as analytes employing six different concentrations to yield sensorgrams for the analysis of association and dissociation kinetics (Figure 5). The sensorgrams were analyzed by applying a 1:1 Langmuir-type inter action model revealing an apparent K_{D} value of 183 ± 42 pM for the binding of wild-type (non-modified/conjugated, not harboring the affinity-enhancing mutation F82D) IL-4 to IL-4Rα Glyco-engineered IL-4 F82D R121C muteins conjugated to different glycan moieties exhibited very high-affinity binding to IL-4Rα, which exceeded that of wildtype IL-4 and Pitrakinra by about 9 to 10fold (Table 2).

**Table 2: Binding affinities of glyco-engineered IL-4 F82D R121C muteins to IL-4Rα ECD in SPR. Kinetic rate and equilibrium binding constants were derived from three independent experiments (n=3) using five different analyte concentrations. The binding parameters kₒₙ, k_{off}, and K_{D} represent mean values and standard deviations.**

| **IL-4 mutein** | **kₒₙ[M⁻¹s⁻¹] x 10⁶** | **k_{off}[s⁻¹] x 10⁻⁴** | **K_{D}[pM]** |
|---|---|---|---|
| WT | 5.34 ± 1.49 | 9.37 ± 0.25 | 183.33 ± 41.68 |
| F82D N38C-Glc | 15.53 ± 2.90 | 2.90 ± 1.57 | 18.67 ± 0.99 |
| F82D R121C-SMCC-GlcN | 4.58 ± 0.58 | 4.18 ± 0.62 | 93.37 ± 25.87 |
| F82D R121C-4acGlc | 7.28 ± 0.82 | 2.57 ± 0.39 | 35.23 ± 3.72 |
| F82D R121C-Glc | 6.99 ± 5.99 | 1.15 ±0.98 | 16.60 ± 0.44 |
| R121D-Y124D | 5.97 ± 0.54 | 9.59 ± 1.45 | 163.33± 39.11 |

The much stronger binding is mainly attributable to reduced dissociation rates (k_{off}) due to the F82D mutation. For disulphide-linked glucose- and glucose tetraacetate-modified IL-4 F82D R121C muteins a K_{D} of about 17 and 35 pM could be determined. The glyco-engineered mutein IL4 F82D R121C coupled to glucosamine via the cross-linker SMCC however exhibited a lower affinity (K_{D} about 90 pM) only two-fold better than wildtype IL-4 despite carrying the same IL-4Rα affinity-enhancing mutation F82D. This lower affinity, in particular when compared to the glycan-conjugates with disulphide-bonded thioglycans, might indicate a lower percentage of active IL-4 protein, which could possibly be due to a harsher treatment during coupling.

### Example 5: Glycosylated IL-4 F82D R121C muteins are highly effective IL-4 antagonists in cell based assays

The biological activity of the glycan-conjugated IL-4 F82D R121C muteins was assessed *in vitro* using two different cell-based experiments. So-called HEK-Blue™ IL-4/IL-13 cells (Invivogen) are stable HEK293 cell transfectants that carry a secreted alkaline phosphatase (SEAP) as reporter gene coupled to an IL-4/IL-13 responsive promoter. As HEK-Blue cells carry only the IL-4 type II receptor comprising IL-4Rα and IL-13Rα1, they serve as a cellular reporter system specific for IL-4 receptor type II signaling. The human erythroleukemia cell line TF-1 also proliferates in response to IL-4 and IL-13 (Lefort et al. 1995). In contrast to HEK-Blue cells, TF1 cells carry both the γc and IL-13Rα1 receptor in addition to IL-4Rα (expression of all three IL-4/IL-13 receptors, i.e. IL-4Rα, γc and IL-13Rα1 were confirmed by quantitative real-time PCR) and therefore can be used to analyze type I and type II receptor signaling experiment. Dose-dependent proliferation stimulated by IL-4 and its glycan-conjugates was quantitatively assessed photometrically by resazurin staining. Both cell lines demonstrated high sensitivity for IL-4 with half-maximal effective concentration (EC₅₀) values of 4 pM for wild-type IL-4 (IL-4 WT) in HEK cells and 7 pM for wild-type IL-4 in TF-1 cells, which is in agreement with published data (Tony et al. 1994, Duppatla et al. 2014).

At a concentration of 50 pM, which is approximately 10-fold higher than the half-maximal effective concentration (EC₅₀) of wild-type IL-4, none of the glyco-engineered IL-4 F82D R121C muteins, independent whether the muteins were conjugated to SMCC glucosamine or disulphide-linked to glucose or tetraacetate glucose, exhibited any biological activity (<1%) in either HEK Blue or TF-1 cells, similar to Pitrakinra, which was used as reference (IL-4 R121D Y124D) (Figure 6). The IL-4 variant R121E fully abrogated IL-4 receptor type II signaling (Shanafeld et al. 1998), but could still efficiently stimulate proliferation of TF-1 cells (maximal proliferation level was 72.7 ± 12.3% compared with wild-type IL-4) consistent with IL-4 R121E being reported as IL-4 receptor type II-specific antagonist. The IL-4 variant F82D N38C was used as negative control. Position 38 is the native N-glycosylation site in IL-4; hence modification with thio-glycans should not interfere with IL-4 activity if it occurs site-specific. In our tests IL-4 F82D N38C conjugated to glucose indeed exhibited biological activities identical to wild-type IL-4, thereby confirming that the production scheme employing refolding in the presence of thio-glycans specifically conjugates to the unpaired cysteine residues and hence does not alter IL-4's native disulphide bonding pattern.

To evaluate the antagonistic efficacy of the glyco-engineered IL-4 F82D R121C muteins, the half-maximal inhibitory concentration (IC₅₀) of each variant was determined from competition of wild-type IL-4 activity using the TF1 cell proliferation assay and by measuring the dose-dependent inhibition of IL-4 induced expression of secreted alkaline phosphatase in HEK-Blue cells. For qualitative analysis and calculation of the inhibitory constant Kᵢ, the EC₅₀ value for wild-type IL-4 was determined first, stimulation of the cells with IL-4 was then done employing the concentration corresponding to the EC₅₀ value. IC₅₀ values were then determined from dose response curves (see Figure 7), the inhibitory constant (Kᵢ) was then calculated using the Cheng-Prusoff equation (Ki = IC₅₀/(1 + [IL-4]/EC₅₀).

The inhibitory constant (Kᵢ) can be considered as a measure of the binding affinity of an antagonist for its receptor, in this case the glyco-engineered IL-4 F82D R121C muteins for the IL-4Rα receptor. The glucose and tetraacetate glucose IL-4 conjugates exhibited a Kᵢ of about 20 pM in TF-1 and HEK-Blue cells, which is more than 5 times lower than the Kᵢ determined for the original Pitrakinra (Kᵢ ≈ 100 pM) (Table 3). The IL-4 variant coupled to SMCC-glucosamine had a slightly higher Kᵢ of about 50 pM, which correlates with its lower binding affinity to IL-4Rα determined in the SPR experiments. These data clearly demonstrate that our glyco-engineered IL-4 F82D R121C muteins block both IL-4 receptors, type I and type II, and therefore present highly effective full IL-4 receptor antagonists that are more potent than the original Pitrakinra (Figure 7 and Table 3).

**Table 3: Inhibitory constants of the glyco-engineered IL-4 F82D R121C variants in vitro. The Kᵢ values were calculated using the IC50 values of the IL-4 F82D R121C muteins and the EC50 value of IL-4 WT (TF-1: 6.7 ± 1.5 pM; HEKBlue: 3.6 ± 1.0 pM) obtained from dose response curves in TF-1 and HEKBlue cell assays as detailed under experimental procedures. Data was used from three independent experiments (n=3).**

| **IL-4 mutein** | **Inhibitory constant (ki) [pM]** | |
|---|---|---|
| | TF-1 | HEKBlue |
| **F82D R121C-SMCC-GlcN** | 51.2 ± 10.4 | |
| **F82D R121C-Glc** | 17.4 ± 6.4 | 13.1 ± 4.1 |
| **F82D R121C-4ac Glc** | 14.6 ± 6.3 | |
| **R121D Y124D** | 98.3 ± 21.3 | 82.4 ± 20.7 |

### Example 6: Hyper-glycosylated IL-4 muteins for extended protein stability

One of the pharmacokinetic disadvantages of the original Pitrakinra is its short serum half-life, which was estimated to about 3 hours. *In vivo,* protein half-life is determined by several biochemical factors such as N-terminal amino acid type (N-end rule), presence of protein degradation motifs (PEST sequences), presence of N- or O-glycosylation, which modulate endocytosis and intracellular protein degradation processes, or by physical factors such as protein size that affects half-life due to clearance via renal filtration. Its small size of only 15 kDa and its lack of glycosylation are certainly key factors determining Pitrakinra's very short serum half-life and therefore severely limited its efficacy as IL-4 inhibitor *in vivo.* It is thus not surprising that attempts were made to improve the pharmacokinetics of Pitrakinra by increasing its size through site-specific conjugation of a 40 kDa PEG polymer. While this indeed improved serum half-life from a few hours to about 5 days, the very long chain length of the linear PEG moiety critically affected IL-4Rα binding and thus decreased its efficacy to act as IL-4 antagonist.

We therefore tested whether a glycosylated IL-4 mutein that is generated by expression in an eukaryotic expression system can be combined with our chemical glycosylation to then provide an IL-4 antagonist with enhanced pharmacokinetic properties due to a complex human-like glycosylation. Several potential N-glycosylation sites were constructed at different positions in IL-4 by altering the amino acid sequence of IL-4 to comprise the N-glycosylation motif N-X-S/T with X not being proline. The IL-4 muteins were then tested whether these novel sites are modified/glycosylated upon recombinant expression in HEK 293 cells. Five IL-4 single amino acid muteins with the substitutions Q20N, E26N, T28N, A34N and K61N were generated showing a mobility shift in SDS-PAGE analysis thereby indicating that the engineered glycosylation sites are modified accordingly (Figure 8). From this set of IL-4 muteins with additional N-glycosylation sites, we designed an IL-4 mutein that comprised three additional N-glycans in order to significantly increase the size of the IL-4 protein. From the above-listed positions the muteins modifying position 20, 28 and 61 (Q20N, T28N and K61N) were chosen, because the single amino acid muteins showed increased expression rate compared to wild-type IL-4, while some other muteins, e.g. E26N, showed strongly decreased protein expression.

SDS-PAGE analysis revealed an apparent molecular weight between 25 and 35 kDa for IL-4 mutein F82D Q20N T28N K61N (Figure 8), which is about a two-fold increase in molecular weight compared to *E. coli* derived wild-type IL-4 or Pitrakinra. To test whether the N-glycosylation affects binding to IL-4Rα, an *in vitro* interaction analysis was performed. The IL-4 mutein F82D Q20N T28N K61N R121C-Glc, which carries complex N-glycans at positions 38 (natural N-glycosylation site of IL-4), 20, 28 and 61, and a disulphide conjugated glucose at position R121C bound to IL-4Rα with an affinity of about 218 ± 28 pM. The 10 times lower affinity compared to the E. coli-derived IL-4 antagonist F82D R121C-Glc (K_{D} about 20 pM) is likely to the significantly increased molecular size, which affects the association rate constants thereby decreasing binding affinity (kₒₙ: 1.12 ± 0.4[M⁻¹s⁻¹] x 10⁶/ k_{off}: 2.37 ± 0.5[s⁻¹] x 10⁻⁴). However, due to rescue by the F82D mutation the binding affinity is not significantly different from wildtype IL-4 (K_{D}: 183 ± 42) and Pitrakinra (K_{D}: 163 ± 39pM) (p > 0.05). In competition with wildtype IL-4 in a TF-1 cell assay the glycosylated IL-4 variant F82D Q20N T28N K61N R121C-Glc attenuated IL-4 dependent response below background level and exhibited a Kᵢ of about 184 ± 55 pM, which is agreement with its K_{D} value.

### Example 7: IL-4 muteins with additional N-glycosylation have enhanced proteolytic stability

Several studies have reported that glycosylation can confer stability against proteolytic degradation by masking proteolytically sensitive sites in a protein. We therefore analyzed if the hyper glycosylated IL-4 muteins also show increased proteolytic stability *in vitro.* IL-4 proteins expressed in different expression systems and carrying different amounts of glycan structures were incubated with bovine trypsin, which here served as model protease. Extended treatment of bacterially derived IL-4 protein with trypsin resulted in complete hydrolysis. Proteolysis was monitored by SDS-PAGE analysis of samples taken at different incubation times of the reaction mixture. Proteolysis progress was then quantified by measuring the Coomassie staining intensity of the protein bands of residual non-proteolyzed IL-4 protein using the Biorad ImageLab software. The "proteolytic" half-life of each IL-4 mutein was determined by regression analysis of the data applying a one phase decay and using the software Prism (Table 4).

**Table 4: Comparison of proteolytic stability of differentially glycosylated IL-4 muteins derived from eukaryotic expression systems. Tryptic degradation of IL-4 muteins over time was analysed using SDS-PAGE as detailed under experimental procedure. Half-life ± standard deviation values were derived from two independent experiments (n=2).**

| IL-4 mutein | Amount of N-glycans | Half-life [h] |
|---|---|---|
| F82D (*E.coli*) | 0 | 0.9 ± 0.05 |
| F82D N38Q (Hek293) | 0 | 2.5 ± 0,1 |
| F82D (Hek293) | 1 | 4.7 ± 1.7 |
| F82D Q20N (Hek293) | 2 | 12.1± 1.0 |
| F82D Q20N-T28N-K61N (Hek293) | 4 | 30.1 ± 1.5 |
| WT (*P. pastoris*) | 1 | 2.4 ± 0,1 |
| WT (Hek293) | 1 | 3.6 ± 0,7 |

Initial experiments showed that the mutations F82D and R121C required to generate the novel IL-4 muteins did not alter proteolytic sensitivity compared to wild-type IL-4. *E*. *coli*-derived IL-4 muteins such as IL-4 F82D or Pitrakinra (IL-4 R121D Y124D), which completely lack glycosylation, were most susceptible to proteolytic degradation by trypsin and exhibited a half-life of only about 1 h under the conditions tested. In contrast, IL-4 F82D mutein obtained from expression in HEK 293 cells, which carries one complex N-glycan at IL-4's natural glycosylation site Asn38 exhibited a half-life of about 5 h showing that the presence of one glycan moiety drastically improved proteolytic stability. To investigate if the enhanced proteolytic stability observed for HEK 293-derived IL-4 is indeed due to glycosylation and not enhanced protein folding stability, an IL-4 mutein N38Q lacking all glycosylation was produced in HEK 293 cells. This mutein IL-4 F82D N38Q showed a half-life of about 2.5 h, indicating that IL-4 mutein expressed in HEK 293 cells is indeed more stable in part certainly due the glycosylation non-existent in *E. coli*-derived IL-4 protein. Why however, the non-glycosylated IL-4 derived from eukaryotic expression exhibits nevertheless a 2-fold longer resistance against hydrolysis by trypsin despite lacking glycosylation as the *E. coli*-derived counterpart remains unclear. Conformingly, IL-4 variants harboring two (Q20N and the natural site at Asn38) or four N-glycosylated sites (Q20N-T28N-K61N and the natural site Asn38) showed a significantly enhanced stability against proteolytic degradation and exhibited half-lives of about 12 and 30 h, respectively, clearly indicating that proteolytic stability of IL-4 can be drastically increased by hyper glycosylation using HEK 293 cells as expression system.

### Example 8: IL-4 mutein F82D K117C may be an IL-4 receptor type I and II antagonist or an IL-4 receptor type II antagonist but a partial IL-4 receptor type I agonist

The IL-4 muteins have been produced as described above. The *E. coli*-derived IL-4 mutein F82D K117C coupled to glutathione (IL4 F82D K117C GSH) fully blunts IL-4 signaling with a Kᵢ of 17 pM, the modified IL-4 antagonist Pitrakinra (carries the mutation F82D for enhanced efficacy) shown as a reference and benchmark exhibits a Kᵢ of about 12 pM in the same assay (Fig. 9A). HEKBlue cells do not carry the IL4 receptor subunit yc, but only IL13Rα1. Hence type II receptor-specific antagonists and full IL-4 antagonist blocking both IL-4 type I and type II receptor activation cannot be distinguished in this assay. Since the same mutein however shows high proliferative activity (resazurin-based measurement of NADH/H* synthesis correlating metabolic activity with proliferation) in TF-1 cells (Figure 9B), which carry both IL-4 receptor types (type I (IL4 specific) and type II (activated by IL-4 and IL-13)), it clearly confirms that IL-4 mutein F82D K117C-GSH is a antagonist specific for IL-4 type II receptor.

To compare a set of IL-4 muteins, two different assays showing their capacity to antagonize IL4 signaling in either TF1 cells (carry IL4 type I as well as type II receptors) and/or to inhibit IL4-induced reporter gene expression via the IL4 type II receptor in HEK Blue cells were performed (Fig. 9B). While IL-4 full antagonists like IL-4 mutein F82D/R121C-GSH, IL-4 mutein F82D/R121C-Glc (*E*. *coli*-derived or the mammalian cell-expression derived IL-4 F82D/R121C-Glc Hek293), IL-4 mutein F82D/R121C-4acGlc (glucose-tetraacetate), IL-4 mutein F82D/R121C-SMCC-Glc (glucosamine coupled via a bifunctional chemical crosslinker) or the 3^{rd} generation IL-4 antagonist IL-4 mutein Q20N/T28N/K61N/F82D/R121C-Glc abrogate IL-4 signaling in both marker cells like the benchmark Pitrakinra, type II receptor antagonists like IL-4 mutein F82D/R121E, or the novel IL4 mutein F82D/T28N (carrying a biosynthetically introduced complex N-glycan at position 28) only shutdown signaling in HEKBlue cells not carrying the yc receptor subunit of the IL4 type I receptor. The latter variant however showed residual IL-4 activity in a couple of experiments and might therefore be also only a partial type II receptor-specific antagonist. A number of IL-4 muteins showed partial type II receptor antagonism, e.g. IL4 F82D/K117C-GSH, IL4 F82D/K117C-4acGlc, showing in comparison with the new type II receptor-specific IL-4 antagonist modified at position 117 that the choice of a modification of the right architecture and size can convert these IL-4 muteins either into IL4 type II receptor antagonists or even IL-4 full antagonists (see IL-4 mutein F82D/N38Q/K117N, lane 5 of the bar diagram).

### REFERENCES

Abbas, A. K.; Murphy, K. M.; Sher, A., Functional diversity of helper T lymphocytes. Nature 1996, 383 (6603), 787-793.
Andrews, A.-L.; Holloway, J. W.; Puddicombe, S. M.; Holgate, S. T.; Davies, D. E., Kinetic analysis of the interleukin-13 receptor complex. The Journal of biological chemistry 2002, 277 (48), 46073-46078.
Barranco, P.; Phillips-Angles, E.; Dominguez-Ortega, J.; Quirce, S., Dupilumab in the management of moderate-to-severe asthma: the data so far. Therapeutics and clinical risk management 2017, 13, 1139-1149.
Bernardes, G. J. L.; Gamblin, D. P.; Davis, B. G., The direct formation of glycosyl thiols from reducing sugars allows one-pot protein glycoconjugation. Angewandte Chemie (International ed. in English) 2006, 45 (24), 4007-4011.
Bochner, B. S.; Klunk, D. A.; Sterbinsky, S. A.; Coffman, R. L.; Schleimer, R. P., IL-13 selectively induces vascular cell adhesion molecule-1 expression in human endothelial cells. Journal of immunology (Baltimore, Md. : 1950) 1995, 154 (2), 799-803.
Carr, C.; Aykent, S.; Kimack, N. M.; Levine, A. D., Disulfide assignments in recombinant mouse and human interleukin 4. Biochemistry 2002, 30 (6), 1515-1523.
Duppatla, V.; Gjorgjevikj, M.; Schmitz, W.; Hermanns, H. M.; Schäfer, C. M.; Kottmair, M.; Muller, T.; Sebald, W., IL-4 analogues with site-specific chemical modification at position 121 inhibit IL-4 and IL-13 biological activities. Bioconjugate chemistry 2014, 25 (1), 52-62.
Duppatla, V.; Gjorgjevikj, M.; Schmitz, W.; Kottmair, M.; Mueller, T. D.; Sebald, W., Enzymatic deglutathionylation to generate interleukin-4 cysteine muteins with free thiol. Bioconjugate chemistry 2012, 23 (7), 1396-1405.
Duschl, A.; Muller, T.; Sebald, W., Antagonistic mutant proteins of interleukin-4. Behring Institute Mitteilungen 1995, (96), 87-94.
Gamblin, D. P.; Garnier, P.; van Kasteren, S.; Oldham, N. J.; Fairbanks, A. J.; Davis, B. G., Glyco-SeS: selenenylsulfide-mediated protein glycoconjugation--a new strategy in post-translational modification. Angewandte Chemie (International ed. in English) 2004, 43 (7), 828-833.
Gandhi, N. A.; Pirozzi, G.; Graham, N. M. H., Commonality of the IL-4/IL-13 pathway in atopic diseases. Expert review of clinical immunology 2017, 13 (5), 425-437.
Graber, P.; Gretener, D.; Herren, S.; Aubry, J. P.; Elson, G.; Poudrier, J.; Lecoanet-Henchoz, S.; Alouani, S.; Losberger, C.; Bonnefoy, J. Y.; Kosco-Vilbois, M. H.; Gauchat, J. F., The distribution of IL-13 receptor alpha1 expression on B cells, T cells and monocytes and its regulation by IL-13 and IL-4. European journal of immunology 1998, 28 (12), 4286-4298.
Hershey, G. K. K., IL-13 receptors and signaling pathways: an evolving web. The Journal of allergy and clinical immunology 2003, 111 (4), 677-90; quiz 691.
Kaur, D.; Hollins, F.; Woodman, L.; Yang, W.; Monk, P.; May, R.; Bradding, P.; Brightling, C. E., Mast cells express IL-13R alpha 1: IL-13 promotes human lung mast cell proliferation and Fc epsilon RI expression. Allergy 2006, 61 (9), 1047-1053.
Kraich, M.; Klein, M.; Patiño, E.; Harrer, H.; Nickel, J.; Sebald, W.; Mueller, T. D., A modular interface of IL-4 allows for scalable affinity without affecting specificity for the IL-4 receptor. BMC biology 2006, 4, 13.
Kruse, N.; Tony, H. P.; Sebald, W., Conversion of human interleukin-4 into a high affinity antagonist by a single amino acid replacement. The EMBO journal 1992, 11 (9), 3237-3244.
Kruse, N.; Shen, B. J.; Arnold, S.; Tony, H. P.; Muller, T.; Sebald, W., Two distinct functional sites of human interleukin 4 are identified by variants impaired in either receptor binding or receptor activation. The EMBO journal 1993, 12 (13), 5121-5129.
LaPorte, S. L.; Juo, Z. S.; Vaclavikova, J.; Coif, L. A.; Qi, X.; Heller, N. M.; Keegan, A. D.; Garcia, K. C., Molecular and structural basis of cytokine receptor pleiotropy in the interleukin-4/13 system. Cell 2008, 132 (2), 259-272.
Leonard, W. J., Cytokines and immunodeficiency diseases. Nature reviews. Immunology 2001, 1 (3), 200-208.
Lefort, S.; Vita, N.; Reeb, R.; Caput, D.; Ferrara, P., IL-13 and IL-4 share signal transduction elements as well as receptor components in TF-1 cells. FEBS letters 1995, 366 (2-3), 122-126.
Longphre, M. V.; Fuller, R. W., Aerovant (recombinant human interleukin-4 variant). In New Drugs and Targets for Asthma and COPD, Hansel, T. T.; Barnes, P. J., Eds. KARGER: Basel, 2010; Vol. 39, pp 123-126.
Muller, T.; Sebald, W.; Oschkinat, H., Antagonist design through forced electrostatic mismatch. Nature Structural & Molecular Biology 1994, 1 (10), 674-676.
Nakamura, T.; Lee, R. K.; Nam, S. Y.; Podack, E. R.; Bottomly, K.; Flavell, R. A., Roles of IL-4 and IFN-gamma in stabilizing the T helper cell type 1 and 2 phenotype. Journal of immunology (Baltimore, Md. : 1950) 1997, 158 (6), 2648-2653.
Parronchi, P.; Carli, M. d.; Manetti, R.; Simonelli, C.; Sampognaro, S.; Piccinni, M. P.; Macchia, D.; Maggi, E.; Del Prete, G.; Romagnani, S., IL-4 and IFN (alpha and gamma) exert opposite regulatory effects on the development of cytolytic potential by Th1 or Th2 human T cell clones. Journal of immunology (Baltimore, Md. : 1950) 1992, 149 (9), 2977-2983.
Punnonen, J.; Aversa, G.; Cocks, B. G.; McKenzie, A. N.; Menon, S.; Zurawski, G.; Waal Malefyt, R. d.; Vries, J. E. d., Interleukin 13 induces interleukin 4-independent IgG4 and IgE synthesis and CD23 expression by human B cells. Proceedings of the National Academy of Sciences of the United States of America 1993, 90 (8), 3730-3734.
Schleimer, R. P.; Sterbinsky, S. A.; Kaiser, J.; Bickel, C. A.; Klunk, D. A.; Tomioka, K.; Newman, W.; Luscinskas, F. W.; Gimbrone, M. A.; Mclntyre, B. W., IL-4 induces adherence of human eosinophils and basophils but not neutrophils to endothelium. Association with expression of VCAM-1. Journal of immunology (Baltimore, Md. : 1950) 1992, 148 (4), 1086-1092.
Serfling R., Coin I., Incorporation of Unnatural Amino Acids into Proteins Expressed in Mammalian Cells. Methods Enzymol 2016, 580, 89-107
Shanafelt, A. B.; Forte, C. P.; Kasper, J. J.; Sanchez-Pescador, L.; Wetzel, M.; Gundel, R.; Greve, J. M., An immune cell-selective interleukin 4 agonist. Proceedings of the National Academy of Sciences of the United States of America 1998, 95 (16), 9454-9458.
Stone, K. D.; Prussin, C.; Metcalfe, D. D., IgE, mast cells, basophils, and eosinophils. The Journal of allergy and clinical immunology 2010, 125 (2 Suppl 2), S73-80.
Tony, H. P.; Shen, B. J.; Reusch, P.; Sebald, W., Design of human interleukin-4 antagonists inhibiting interleukin-4-dependent and interleukin-13-dependent responses in T-cells and B-cells with high efficiency. European journal of biochemistry 1994, 225 (2), 659-665.
van Kasteren, S. I.; Kramer, H. B.; Gamblin, D. P.; Davis, B. G., Site-selective glycosylation of proteins: creating synthetic glycoproteins. Nature protocols 2007, 2 (12), 3185-3194.
Wenzel, S.; Wilbraham, D.; Fuller, R.; Getz, E. B.; Longphre, M., Effect of an interleukin-4 variant on late phase asthmatic response to allergen challenge in asthmatic patients: results of two phase 2a studies. Lancet (London, England) 2007, 370 (9596), 1422-1431.
Worm, M.; Henz, B. M., Molecular regulation of human IgE synthesis. Journal of molecular medicine (Berlin, Germany) 1997, 75 (6), 440-447.
Xia, H. Z.; Du, Z.; Craig, S.; Klisch, G.; Noben-Trauth, N.; Kochan, J. P.; Huff, T. H.; Irani, A. M.; Schwartz, L. B., Effect of recombinant human IL-4 on tryptase, chymase, and Fc epsilon receptor type I expression in recombinant human stem cell factor-dependent fetal liver-derived human mast cells. Journal of immunology (Baltimore, Md. : 1950) 1997, 159 (6), 2911-2921.
Zhu, Z.; Homer, R. J.; Wang, Z.; Chen, Q.; Geba, G. P.; Wang, J.; Zhang, Y.; Elias, J. A., Pulmonary expression of interleukin-13 causes inflammation, mucus hypersecretion, subepithelial fibrosis, physiologic abnormalities, and eotaxin production. The Journal of clinical investigation 1999, 103 (6), 779-788.

## Claims

1. A IL-4 mutein capable of inhibiting IL-4 and/or IL-13 signalling, wherein the IL-4 mutein comprises an amino acid mutation at each of sequence positions 82 and 121 in comparison to the human mature IL-4 (positions 25-153 of UniProt database entry P05112, SEQ ID NO: 1), wherein the amino acid at sequence position 82 is mutated to aspartic acid and the amino acid at sequence position 121 is mutated to an amino acid, which allows site-directed conjugation of a non-protein-polymer, and wherein a non-protein-polymer is conjugated to the mutein via the mutated amino acid at sequence position 121.

2. An IL-4 mutein capable of inhibiting IL-4 and/or IL-13 signalling, wherein the IL-4 mutein comprises an amino acid mutation at each of sequence positions 82 and 117 in comparison to the human mature IL-4 (positions 25-153 of UniProt database entry P05112, SEQ ID NO: 1), wherein the amino acid at position 82 is mutated to aspartic acid and the amino acid at position 117 is mutated to an amino acid, which allows site-directed conjugation of a non-protein-polymer, and wherein a non-protein-polymer is conjugated to the IL-4 mutein via the mutated amino acid of position 117.

3. The IL-4 mutein of claim 2, wherein the IL-4 mutein comprises a further mutation at position 124, wherein the amino acid at position 124 is mutated to an amino acid selected from the group consisting of glycine, lysine and asparagine.

4. The IL-4 mutein of any of the preceding claims, further comprising at least one mutated amino acid at a sequence position selected from the group consisting of sequence positions 20, 28 and 61.

5. The IL-4 mutein of claim 4, wherein the at least one mutated amino acid(s) at a sequence position selected from the group consisting of sequence positions 20, 28 and 61 provides one additional glycosylation site at each mutated sequence position, or is mutated to an amino acid, which allows site-direct conjugation of a non-protein polymer and wherein a non-protein polymer is conjugated to the IL-4 mutein via the mutated amino acid at a sequence position selected from the group consisting of positions 20, 28 and 61

6. The IL-4 mutein of any of the preceding claims, further comprising a mutated amino acid at sequence position 38, wherein the amino acid at sequence position 38 is mutated to an amino acid, which allows site-directed conjugation of a non-protein-polymer and wherein a non-protein polymer is conjugated to the IL-4 mutein via the mutated amino acid of sequence position 38.

7. The IL-4 mutein of any of the preceding claims, comprising an amino acid sequence with at least 75 %, at least 80 %, at least 85 %, at least 90 %, at least 95 % or at least 99 % sequence identity to an amino acid sequence selected from the group consisting of SEQ ID NOs: 2-10, wherein the IL-4 mutein comprises at least one amino acid, which allows site-directed conjugation of a non-protein polymer and wherein a non-protein polymer is conjugated to the IL-4 mutein via the amino acid, which allows site-directed conjugation of a non-protein polymer.

8. The IL-4 mutein of any of the preceding claims, wherein the amino acid, which allows site-directed conjugation of a non-protein-polymer, is cysteine.

9. The IL-4 mutein of any of the preceding claims, wherein the non-protein polymer comprises a mono- or oligosaccharide, polysialic acid or a complex N-glycan-like oligosaccharide structure.

10. The IL-4 mutein of any of the preceding claims, wherein the IL-4 mutein has a glycosylation pattern obtained by an expression in a eukaryotic cell.

11. A nucleic acid encoding the IL-4 mutein of any one of claims 1-10.

12. A method for producing an IL-4 mutein according to any of claims 1 to 10, comprising the steps of:
(a) culturing the host cell comprising the nucleic acid of claim 11 under conditions allowing the production of the IL-4 mutein,
(b) obtaining the IL-4 mutein,
(c) optionally conjugating the IL-4 mutein with a non-protein polymer,
(d) optionally removing non-conjugated IL-4 muteins, and
(e) optionally enzymatic elongation of chemically coupled mono- and/or oligosaccharides.

13. A pharmaceutical composition comprising the IL-4 mutein of any one of claims 1-10.

14. The IL-4 mutein of any of claims 1-10 or the pharmaceutical composition of claim 13 for use in a method of treating a subject.

15. The IL-4 mutein of any of claims 1-10, the pharmaceutical composition of claim 13 for use in a method of treating leishmaniosis or an inflammatory disease, preferably the inflammatory disease is selected from the list consisting of asthma, arthritis, colitis ulcera, atopic dermatitis, allergy and allergic asthma.
